# EUROPÄISCHE PATENTSCHRIFT

(11) **EP 2 930 126 B1**
(45) Veröffentlichungstag und Bekanntmachung des Hinweises auf die Patenterteilung: **09.10.2019**
(21) Anmeldenummer: 15155751.9
(22) Anmeldetag: 19.02.2015
(51) Int. Cl.: B65D 83/14, B65D 83/48, A61K 8/04, A61K 8/29, A61K 8/26, A61K 8/31, A61K 8/81, A61Q 5/06

(54) **Modische Aerosol-Färbemittel**
Fashionable aerosol dye
Colorant par aérosol à la mode

(30) Priorität: 04.04.2014 DE 102014206532
(43) Veröffentlichungstag der Anmeldung: 14.10.2015
(73) Patentinhaber: Henkel AG & Co. KGaA, 40589 Düsseldorf (DE)
(72) Erfinder: Goutsis, Konstantin, 41363 Jüchen (DE); Weser, Gabriele, 41472 Neuss (DE)

(56) Entgegenhaltungen:
- EP-A1- 2 062 616
- US-A1- 2009 068 124
- US-A1- 2012 034 173
- Anonymous: "ASPA - Color Make-Up for Hair Product Description", GNPD, 1. November 2011 (2011-11-01), Seiten 1-3, XP055206519, Internet Gefunden im Internet: URL:http://www.gnpd.com/sinatra/recordpage /1637770/from_search/7Wqnrd2DlR/ [gefunden am 2015-08-06]
- Anonymous: "Bumble and Bumble Spraychalk", GNPD, 1. Dezember 2013 (2013-12-01), Seiten 1-3, XP055206521, Internet Gefunden im Internet: URL:http://www.gnpd.com/sinatra/recordpage /2260127/from_search/EPJpHfOZU9/ [gefunden am 2015-08-06]

## Beschreibung

Die Erfindung betrifft Mittel zum Färben keratinischer Fasern, die eine zeitlich befristete Färbung ermöglichen, welche leicht appliziert und ebenso leicht wieder entfernt werden kann.
Bei den üblichen Färbeverfahren wird das Färbemittel in Form einer Creme oder eines Gels mit der Hand oder einem Pinsel auf das Haar aufgetragen. Diese Haarbehandlung ist mit einem verhältnismäßig großen Aufwand verbunden und beeinflusst häufig auch in ungewünschter Weise die Kopfhaut. Des Weiteren ist es entsprechend der zeitgemäßen Mode häufig erwünscht, einzelne Haarsträhnen auf einfache Weise einzufärben, ohne das übrige Haar oder die Kopfhaut zu beeinträchtigen. Diese Färbung soll einerseits unabhängig von der Ausgangshaarfarbe - also insbesondere auch auf dunklem Haar - möglich sein, ohne vorher blondieren zu müssen, andererseits soll sie reversibel sein, d.h. sich bei Bedarf schnell ändern bzw. entfernen lassen.
Hierfür haben sich Tönungen im Markt etabliert, die aber nicht allen Anforderungen gerecht werden. Zum einen ist die Applikation immer noch zeitaufwendig und in einigen Fällen mit Kopfhautirritationen verbunden, zum anderen ist das Einfärben dunkler Ausgangshaarfarben mit helleren Farbtönen nur schwierig realisierbar.
Manchmal besteht der Verbraucherwunsch, nicht nur Strähnen oder das gesamte Haar zu färben, sondern geometrische Muster, Buchstaben, Zeichen o.ä. auf dem Kopf farblich hervorzuheben, z.B. ein grünes Kleeblatt am St. Patrick's Day oder das "Peace"-Zeichen bei Friedendemonstrationen. Solche Färbungen sind nur unter verhältnismäßig großem Aufwand realisierbar.
Im Bereich der Faschingshaarfarben haben sich Aerosole etabliert, welche Pigmente enthalten und so die weitverbreitete Übung, dekorative Kosmetik ("Schminke") im Karneval zur Einfärbung von Haaren zu nutzen, in einem auf die Haare abgestimmten Produkt anbieten. Diese Produkte haben einerseits als Scherzartikel bzw. Karnevalsbedarf oft eine nur geringe Haltbarkeit, neigen zum Abfärben auf die Kleidung und sind oft nur saisonal verfügbar, andererseits fallen sie nicht unter das Kosmetikrecht und bieten dem Verbraucher keine ausreichende Information über Inhaltsstoffe (INCI-Deklaration) sowie keine Pflege oder anderen kosmetischen Zusatznutzen.
Zudem weisen diese Produkte als Wegwerf- und Saisonartikel nur geringe Lagerstabilitäten auf und haben nach der Erstbenutzung Probleme mit dem Verstopfen der Sprühdüsen ("clogging"), der Haltbarkeit auf dem Haar und einem Absetzen des Produktes in der Aerosoldose, was sich in ungleichmäßigen Sprühbildern äußerst, die im Karneval kaum, im normalen Verbraucheralltag aber sehr wohl negativ auffallen.

Entsprechende Produkte sind beispielsweise als "ASPA Color Make-Up for Hair" (Mintel-ID 1637770) oder "Bumble and Bumble Spraychalk" (Mintel-ID 2260127) erhältlich.

Die US 2009/068124 A1 offenbart Zusammensetzungen, die bevorzugt in der Haarpflege wie beispielsweise der temporären Haarfärbung eingesetzt werden.

Aerosolformulierungen zur Haarformung werden in der EP 2 062 616 A1 offenbart.

Die US 2012/043173 A1 offenbart eine Zweikomponenten-Aerosolfarbe, die in einem Aerosoldispenser enthalten ist.

Der vorliegenden Erfindung lag die Aufgabe zugrunde, leicht applizier- und entfernbare Haarfarben bereitzustellen, die frei von den vorstehend genannten Problemen sind. Dabei sollten die Haltbarkeit des Produktes und der mit ihm erzielten Färbung, die Lagerstabilität und die Möglichkeit eines kosmetischen Zusatznutzens im Vordergrund stehen. Zudem sollte das Produkt auch mehrfach ohne Beeinträchtigung der Leistung applizierbar sein.

Es wurde nun gefunden, dass sich sprühbare Pigmenthaarfarben bereitstellen lassen, deren Zusammensetzung den vorstehend genannten Problemkreis in deutlicher Weise löst.

Gegenstand der vorliegenden Erfindung ist in einer ersten Ausführungsform ein Haarbehandlungsmittel, umfassend einen Druckbehälter und - darin befindlich - eine Aerosolzusammensetzung, die - bezogen auf ihr Gewicht-
a) 20 bis 80 Gew.-% mindestens eines Treibmittels;
b) 1 bis 79 Gew.-% mindestens eines Weißpigments;
c) 0,1 bis 50 Gew.-% mindestens eines Viskositätsgebers,
d) 0 bis 1 Gew.-% Farbstoff(e),
e) 0,1 bis 10 Gew.-% Talkum
enthält.

Das erfindungsgemäße Haarbehandlungsmittel umfasst einen Aerosol-Druckbehälter. Als Druckgasbehälter kommen Gefäße aus Metall (Aluminium, Weißblech, Zinn), geschütztem bzw. nichtsplitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der im Aerosol-Druckbehälter enthaltenen Zubereitung.

Besonders gute erfindungsgemäße Effekte werden erzielt, wenn der Innendruck des Aerosol-Druckbehälters bei mindestens 1,8 bar, insbesondere mindestens 2,5 bar, beträgt.

Das Produkt umfasst weiterhin eine Aerosol-Abgabevorrichtung, welche zur Abgabe des Aerosols ein Ventil besitzt. In einer bevorzugten Ausführungsform der Erfindung weist das Ventil einen mit einem Lack oder einem polymeren Kunststoff A beschichteten Ventilteller und ein ebensolches flexibles Element mit Rückstellcharakteristik auf, das das Ventil nach Beenden der Betätigung in die Verschlußstellung (= Ruhelage des Ventils) zurückstellt. Entsprechende erfindungsgemäße Haarbehandlungsmittel, bei denen die Aerosol-Abgabevorrichtung ein Ventil umfasst, das einen Ventilkegel und/oder ein flexibles Element mit Rückstellcharakteristik aufweist, der/das/die mit einem Lack oder einem polymeren Kunststoff A beschichtet ist/sind, sind erfindungsgemäß bevorzugt.

In einer weiteren bevorzugten Ausführungsform der Erfindung weist das Ventil ein flexibles Element mit Rückstellcharakteristik und/oder einen Ventilteller aus mindestens einem Kunststoff B, bevorzugt einem elastomeren Kunststoff, auf. Auch hier sind erfindungsgemäße Haarbehandlungsmittel, bei denen das Ventil ein flexibles Element mit Rückstellcharakteristik und/oder einen Ventilkegel aus mindestens einem Kunststoff B aufweist, bevorzugt, wobei bevorzugte Kunststoffe B elastomere Kunststoffe sind. Besonders bevorzugte elastomere Kunststoffe sind ausgewählt aus Buna, insbesondere Buna N, Buna 421, Buna 1602 und Buna KA 6712, Neopren, Butyl und Chlorbutyl.

In einer weiteren bevorzugten Ausführungsform der Erfindung kann das flexible Element mit Rückstellcharakteristik als Spiralfeder bzw. Schraubendruckfeder ausgebildet sein. In einer weiteren bevorzugten Ausführungsform der Erfindung kann das flexible Element des Ventils mit Rückstellcharakteristik einstückig mit dem Ventilkegel ausgebildet sein und biegsame Beine aufweisen. Diese Feder kann aus Metall oder Kunststoff sein.

In einer besonders bevorzugten Ausführungsform der Erfindung sind Ventilkegel und flexibles Element mit Rückstellcharakteristik ausgebildet. Besonders bevorzugt ist dabei der Ventiltyp Ariane M, erhältlich von der Firma Seaquist Perfect, bei dem das flexible Element mit Rückstellcharakteristik in Form von vier elastischen Beinen einstückig mit dem Ventilkegel ausgebildet ist.

Alle erfindungsgemäß verwendeten Ventile weisen vorzugsweise einen innenlackierten Ventilteller auf, wobei Lackierung und Ventilmaterial miteinander kompatibel sind. Werden erfindungsgemäß Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Die eingesetzten Behälter, die z. B. aus Weißblech oder aus Aluminium sein können, wobei Aluminiumbehälter erfindungsgemäß bevorzugt sind, sollten angesichts der möglichen Korrosivität der erfindungsgemäße Haarbehandlungsmittel ebenfalls innen lackiert oder beschichtet sein. Ein erfindungsgemäß bevorzugter Innenschutzlack ist ein Epoxy-Phenollack, wie er u.a. unter der Bezeichnung Hoba 7407 P erhältlich ist.

Ganz besonders bevorzugt handelt es sich bei dem Ventil um ein Ventil des Typs Aptar ARM-4.00-1-0, 32-8, 70 Green - AR Housing - Valve-AHT-1,60-0,00-PA-Natural.

Das erfindungsgemäße Haarbehandlungsmittel enthält als ersten wesentlichen Bestandteil (a) Dimein Treibmittel oder Treibmittelgemisch.

Erfindungsgemäß bevorzugte Treibmittel (Treibgase) sind ausgewählt aus Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination. Besonders bevorzugt sind Propan, n-Butan, iso-Butan sowie, besonders bevorzugt, Mischungen dieser Treibmittel. Auch hydrophile Treibgase, wie z. B. Kohlendioxid, können vorteilhaft im Sinne der vorliegenden Erfindung eingesetzt werden. Besonders bevorzugt sind Propan, n-Butan, iso-Butan sowie insbesondere Mischungen dieser Treibgase. Die Menge der Treibmittel beträgt 20 bis 80 Gew.-%, vorzugsweise 22,5 bis 77,5 Gew.-%, weiter bevorzugt 25 bis 75 Gew.-%, noch weiter bevorzugt 27,5 bis 72,5 Gew.-% und insbesondere 30 bis 70 Gew.-% Treibmittel oder Treibmittelgemisch, jeweils bezogen auf das Gesamtgewicht der im Druckbehälter befindlichen Zubereitung.

Gemäß einer bevorzugten Ausführungsform enthalten die erfindungsgemäßen Zubereitungen die genannten Kohlenwasserstoffe, Dimethylether oder Mischungen der genannten Kohlenwasserstoffe mit Dimethylether als einziges Treibmittel. Die Erfindung umfaßt aber ausdrücklich auch die Mitverwendung von Treibmittel vom Typ der Fluorchlorkohlenwasserstoffe, insbesondere aber der Fluorkohlenwasserstoffe.

Ganz besonders bevorzugte erfindungsgemäße Haarbehandlungsmittel sind dadurch gekennzeichnet, daß die Aerosolzusammensetzung - bezogen auf ihr Gewicht - 20 bis 80 Gew.-%, vorzugsweise 22,5 bis 77,5 Gew.-%, weiter bevorzugt 25 bis 75 Gew.-%, noch weiter bevorzugt 27,5 bis 72,5 Gew.-% und insbesondere 30 bis 70 Gew.-% Treibmittel oder Treibmittelgemisch, ausgewählt aus Dimethylether, HFO1234yf, HFO1234ze, Propan, Propen, n-Butan, iso-Butan, iso-Buten, n-Pentan, Penten, iso-Pentan und iso-Penten oder deren Mischungen, enthält. Dimethylether führt als Treibmittel (a) zusammen mit den weiteren wesentlichen Formulierungsbestandteilen zu einer optimalen Auffächerung des Sprühstrahls und einer deutlich verringerten Neigung zur Ventilverstopfung ("clogging").

Als zweiten wesentlichen Bestandteil enthält die im Druckbehälter befindliche Zubereitung 1 bis 79 Gew.-% mindestens eines Weißpigments. Weißpigmente sind unbunte anorganische Pigmente mit einem hohen Brechungsindex (idR größer 1,8), die in der Regel synthetisch hergestellt und vor allem zur Erzeugung von optischer Weiße in Anstrichmitteln oder als Füllstoff in z. B. Kunststoffen verwendet werden. Erfindungsgemäß besonders bevorzugt einsetzbar ist vor allem Titandioxid, daneben haben sich Zinkoxid und das für diese technischen Zwecke eher ungebräuchliche Aluminiumstärkeoctenylsuccinat als erfindungsgemäß besonders geeignet erwiesen.

Ganz besonders bevorzugte erfindungsgemäße Haarbehandlungsmittel sind dadurch gekennzeichnet, daß die Aerosolzusammensetzung - bezogen auf ihr Gewicht - 20 bis 80 Gew.-%, vorzugsweise 22,5 - bezogen auf ihr Gewicht - 1,5 bis 75 Gew.-%, vorzugsweise 2 bis 50 Gew.-%, weiter bevorzugt 2,5 bis 40 Gew.-%, noch weiter bevorzugt 3 bis 30 Gew.-%, besonders bevorzugt 3,5 bis 25 Gew.-% und insbesondere 4 bis 15 Gew.-% Weißpigment(e) aus der Gruppe Titandioxid, Zinkoxid, Zinksulfid, Lithopone, Aluminiumstärkeoctenylsuccinat oder deren Mischungen, enthält. Als zweiten wesentlichen Bestandteil enthält die im Druckbehälter befindliche Zubereitung 0,1 bis 50 Gew.-% mindestens eines Viskositätsgebers. Es hat sich gezeigt, dass der Zusatz des Viskositätsgebers die Lagerstabilität der gesamten Zubereitung deutlich verbessert, ohne die Neigung zur Ventilverstopfung ("clogging") zu erhöhen. Darüber hinaus haftet die erfindungsgemäße Zusammensetzung besser auf dem Haar, so dass die unbeabsichtigte Anfärbung anderer Haarpartien oder der Kleidung deutlich minimiert wird.

Ganz besonders bevorzugt sind erfindungsgemäße Haarbehandlungsmittel, bei denen die Aerosolzusammensetzung - bezogen auf ihr Gewicht - 0,25 bis 40 Gew.-%, vorzugsweise 0,5 bis 30 Gew.-%, weiter bevorzugt 0,75 bis 20 Gew.-%, noch weiter bevorzugt 1 bis 15 Gew.-%, besonders bevorzugt 1,25 bis 10 Gew.-% und insbesondere 1,5 bis 7,5 Gew.-% Viskositätsgeber aus der Gruppe der ggf. modifizierten quellfähigen Schichtsilikate, der verdickenden Acrylpolymere oder deren Mischungen, enthält.

Als Viskositätsgeber eignen sich sowohl anorganische als auch organische Stoffe. Bei den anorganischen Stoffen haben sich insbesondere Kieselsäuren und Silikate als besonders geeignet erwiesen. Ganz besonders bevorzugt, weil hervorragend versprühbar und sehr gut haftfähig sind erfindungsgemäße Haarbehandlungsmittel, bei denen die Aerosolzusammensetzung - bezogen auf ihr Gewicht - 0,25 bis 40 Gew.-%, vorzugsweise 0,5 bis 30 Gew.-%, weiter bevorzugt 0,75 bis 20 Gew.-%, noch weiter bevorzugt 1 bis 15 Gew.-%, besonders bevorzugt 1,25 bis 10 Gew.-% und insbesondere 1,5 bis 7,5 Gew.-% Viskositätsgeber aus der Gruppe der ggf. modifizierten Smektite, vorzugsweise aus der Gruppe der ggf. modifizierten Montmoriilonite und/oder der ggf. modifizierten Hectorite enthält, wobei das Reaktionsprodukt von N,N-Dimethyl-N-octadecyl-1-Octadecanaminiumchlorid mit Hectorit (INCI-Bezeichnung: DISTEARDIMONIUM HECTORITE) besonders bevorzugt ist.

Unter den organischen Viskositätsgebern können sowohl natürliche Stoffe als auch synthetische Viskositätsgeber eingesetzt werden. Natürliche Stoffe sind beispielsweise Polysaccharide wie Stärke, Pektin, Agarose, Gummiarabicum und Johannisbrotkernmehl. Auch Polypeptide wie Gelatine und Casein wirken durch die Ausbildung von Wasserstoff-Brückenbindungen etc. als Verdickungsmittel. Auch modifizierte Naturstoffe wie Carnboxymethylcellulose oder -stärke sind erfindungsgemäß geeignet.

Vollsysnthetische Viskositätsgeber können Polymere aus den unterschiedlichsten Monomeren sein, wobei sich Acrylatpolymere, also Homo- oder Copolymere der Acrylsäure oder Methacrylsäure, ihren jeweiligen Salzen oder Estern ganz besonders bewährt haben.

Ganz besonders bevorzugt, weil hervorragend versprühbar und sehr gut haftfähig sind erfindungsgemäße Haarbehandlungsmittel, bei denen die Aerosolzusammensetzung - bezogen auf ihr Gewicht - 0,25 bis 40 Gew.-%, vorzugsweise 0,5 bis 30 Gew.-%, weiter bevorzugt 0,75 bis 20 Gew.-%, noch weiter bevorzugt 1 bis 15 Gew.-%, besonders bevorzugt 1,25 bis 10 Gew.-% und insbesondere 1,5 bis 7,5 Gew.-% Viskositätsgeber aus der Gruppe der verzweigten (Meth-)Acrylate (INCI-Bezeichnung ACRYLATE CROSSPOLYMER) enthält, wobei ein verzweigtes (Co-)Polymer von Methylmethacrylsäure (INCI-Bezeichnung: METHYL METHACRYLATE CROSSPOLYMER) besonders bevorzugt ist.

Um die keratinischen Fasern in der gewünschten Farbe anzufärben, enthalten die erfindungsgemäßen Zubereitungen vorzugsweise Farbstoffe, besonders bevorzugt in Mengen von 0,01 bis 1 Gew.-%, bezogen auf die Aerosolzusammensetzung. Hier können Pigmente eingesetzt werden, die deckend auch zur helleren Färbung dunkler Ausgangshaarfarben geeignet sind, es ist aber auch möglich, lösliche Farbstoffe oder Lösungen von Farbstoffen in die Mittel zu inkorporieren, um "leichtere Töne" direkt auf helleres Haar oder auf die in der Zubereitung enthaltenen Weißpigmente zu färben, die dann das Haar entsprechend farblich modifizieren. In der Praxis haben sich insbesondere Mischungen aus beiden Varianten, d.h. der gleichzeitige Einsatz löslicher Farbstoffe und Pigmente als geeignet erwiesen. Für sehr helle Färbungen auf dunklem Ausgangshaar kann es vorteilhaft sein, als Farbstoff(e) ausschließlich Pigment(e) einzusetzen, wobei in allen Fällen die Menge an Weißpigmenten a) nicht zur Menge der Farbstoffe d) hinzugerechnet wird.

Die erfindungsgemäßen Mittel können mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 1 Gew.-%, bezogen auf die gesamte Anwendungszubereitung, eingesetzt. Direktziehende Farbstoffe können in anionische, kationische und nichtionische direktziehende Farbstoffe unterteilt werden.

Als anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobezolsulfonsäuresäurenatriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure-natriumsalz (C.I. 10,385; Acid Orange 3), 4-[(2,4-Dihydroxyphenyl)azo]-benzolsulfonsäure-natriumsalz (C.I. 14,270; Acid Orange 6), 4-[(2-Hydroxynaphth-1-yl)azo]-benzolsulfonsäure-natriumsalz (C.I. 15,510; Acid Orange 7), 4-[(2,4-Dihydroxy-3-[(2,4-dimethylphenyl)azo]-phenyl)azo]-benzolsulfonsäure-natriumsalz (C.I. 20,170; Acid Orange 24), 4-Hydroxy-3-[(2-methoxyphenyl)azo]-1-naphthalinsulfonsäure-natriumsalz (C.I. 14,710; Acid Red 4), 4-Hydroxy-3-[(4-sulfonaphth-1-yl)azo]-1-naphthalin-sulfonsäure-dinatriumsalz (C.I. 14,720; Acid Red No.14), 6-Hydroxy-5-[(4-sulfonaphth-1-yl)azo]-2,4-naphthalin-disulfonsäure-trinatriumsalz (C.I. 16,255; Ponceau 4R; Acid Red 18), 3-Hydroxy-4-[(4-sulfonaphth-1-yl)azo]-2,7-naphthalin-disulfonsäure-trinatriumsalz (C.I. 16,185; Acid Red 27), 8-Amino-1-hydroxy-2-(phenylazo)-3,6-naphthalin-disulfonsäure-dinatriumsalz (C.I. 17,200; Acid Red 33; Red 33), 5-(Acetylamino)-4-hydroxy-3-[(2-methylphenyl)azo]-2,7-naphthalin-disulfonsäure-dinatriumsalz (C.I. 18,065; Acid Red 35), 2-(3-Hydroxy-2,4,5,7-tetraiod-dibenzopyran-6-on-9-yl)-benzoesäure-dinatriumsalz (C.I. 45,430; Acid Red 51), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 8-[(4-(Phenylazo)phenyl)azo]-7-naphthol-1,3-disulfonsäure-dinatriumsalz (C.I. 27,290; Acid Red 73), 2',4',5',7'-Tetrabrom-3',6'- dihydroxyspiro[isobenzofuran-1(3H),9'-[9H]xanthen]-3-on-dinatriumsalz (C.I. 45,380; Acid Red 87), 2',4',5',7'-Tetrabrom-4,5,6,7-tetrachlor-3',6'-dihydroxyspiro[isobenzofuran-1(3H),9'[9H]xanthen]-3-on-dinatriumsalz (C.I. 45,410; Acid Red 92), 3',6'-Dihydroxy-4',5'-diiodospiro[isobenzofuran-1(3H),9'(9H)- xanthen]-3-on-dinatriumsalz (C.I. 45425; Acid Red 95), 2-Hydroxy-3-((2- hydroxynaphth-1-yl)azo)-5-nitrobenzolsulfonsäure-natriumsalz (C.I. 15,685; Acid Red 184), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red 195), 3-Hydroxy-4-[(4-methyl-2-sulfonphenyl)-azo]-2-naphthalincarbonsäure-calciumsalz (C.I. 15,850:1; Pigment Red 57:1), 3-[(2,4-Dimethyl-5-sulfophenyl)azo]-4-hydroxy-1-naphthalin-sulfonsäure-dinatriumsalz (C.I. 14,700; Food Red No. 1; Ponceau SX; FD&C Red No. 4), 1,4-Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), Bis[4-(diethylamino)-phenyl](2,4-disulfophenyl)carbenium-inneres Salz, Natriumsalz (2:1) (C.I. 42,045; Food Blue No. 3; Acid Blue 1), Bis[4-(diethylamino)phenyl](5-hydroxy-2,4-disulfophenyl)-carbenium-inneres Salz, Calciumsalz (2:1) (C.I. 42,051; Acid Blue 3), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(phenylamino)-9,10-anthrachinon-2-sulfonsäure (C.I. 62,055; Acid Blue 25), 1-Amino-4-(cyclohexylamino)-9,10-anthrachinon-2-sulfonsäure-natriumsalz (C.I. 62045; Acid Blue 62), 2-(1,3-Dihydro-3-oxo-5-sulfo-2H-indol-2-yliden)-2,3-dihydro-3-oxo-1H-indol-5- sulfonsäure-dinatriumsalz (C.I. 73,015; Acid Blue 74), 9-(2-Carboxyphenyl)-3-[(2-methylphenyl)amino]-6-[(2-methyl-4-sulfophenyl)amino]xanthylium-inneres Salz, Natriumsalz (C.I. 45,190; Acid Violet 9), 1-Hydroxy-4-[(4-methyl-2-sulfophenyl)amino]-9,10-anthrachinon-natriumsalz (C.I. 60,730; D&C Violett No. 2; Acid Violet 43), Bis[3-nitro-4-[(4-phenylamino)-3-sulfo-phenylamino]-phenyl]-sulfon (C.I. 10,410; Acid Brown 13), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440; Food Black No. 1), 3',3",5',5"-Tetrabromphenolsulfonphthalein (Bromphenolblau), 3,4,5,6,3',3",5',5"-Octabromphenolsulfonphthalein (Tetrabromphenolblau).

Bevorzugte anionische direktziehende Farbstoffe sind die unter den internationalen Bezeichnungen bzw. Handelsnamen Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Als kationische direktziehende Farbstoffe eignen sich insbesondere 9-(Dimethylamino)-benzo[a]phenoxazin-7-ium-chlorid (C.I. 51,175; Basic Blue 6), Di[4-(diethylamino)phenyl][4-(ethylamino)naphthyl]carbenium-chlorid (C.I. 42,595; Basic Blue 7), Di-(4-(dimethylamino)phenyl)-(4-(methyl-phenylamino)-naphthalin-1-yl)carbenium-chlorid (C.I. 42,563; Basic Blue 8), 3,7-Di(dimethylamino)phenothiazin-5-ium-chlorid (C.I. 52,015 Basic Blue 9), Di[4-(dimethylamino)phenyl][4-(phenylamino)naphthyl] carbenium-chlorid (C.I. 44,045; Basic Blue 26), 2-[(4-(Ethyl(2-hydroxyethyl)amino)phenyl)azo]-6-methoxy-3-methyl-benzothiazolium-methylsulfat (C.I. 11,154; Basic Blue 41), 8-Amino-2-brom-5-hydroxy-4-imino-6-[(3-(trimethylammonio)phenyl)amino]-1(4H)-naphthalinon-chlorid (C.I. 56,059; Basic Blue No. 99), Bis[4-(dimethylamino)phenyl]-[4-(methylamino)phenyl]carbenium-chlorid (C.I. 42,535; Basic Violet 1), Tri(4-amino-3-methylphenyl)carbenium-chlorid (C.I. 42,520; Basic Violet 2), Tri[4-(dimethylamino)-phenyl]carbenium-chlorid (C.I. 42,555; Basic Violet 3), 2-[3,6-(Diethylamino)dibenzopyranium-9-yl]- benzoesäurechlorid (C.I. 45,170; Basic Violet 10), Di(4-aminophenyl)(4-amino-3- methylphenyl)carbeniumchlorid (C.I. 42,510 Basic Violet 14), 1,3-Bis[(2,4-diamino-5-methylphenyl)azo]-3-methylbenzol (C.I. 21,010; Basic Brown 4), 1-[(4-Aminophenyl)azo]-7-(trimethylammonio)- 2-naphthol-chlorid (C.I. 12,250; Basic Brown 16), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (C.I. 12,251; Basic Brown 17), 3-[(4-Amino-2,5-dimethoxyphenyl)azo]-N,N,N-trimethylbenzolaminiumchlorid (C.I. 12,605, Basic Orange 69), 3,7-Diamino-2,8-dimethyl- 5-phenylphenazinium-chlorid (C.I. 50,240; Basic Red 2), 1,4-Dimethyl-5-[(4-(dimethylamino)phenyl)azo]-1,2,4-triazolium-chlorid (C.I. 11,055; Basic Red 22), 2-Hydroxy-1-[(2-methoxyphenyl)azo]-7-(trimethylammonio)-naphthalin-chlorid (C.I. 12,245; Basic Red 76), Di[4-(dimethylamino)phenyl]iminomethan-hydrochlorid (C.I. 41,000; Basic Yellow 2), 2-[2-((2,4-Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57), Bis[4-(diethylamino)phenyl]phenylcarbenium-hydrogensulfat (1:1) (C.I. 42,040; Basic Green 1), Di(4- (dimethylamino)phenyl)-phenylmethanol (C.I. 42,000; Basic Green 4), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid und direktziehende Farbstoffe, die einen Heterocyclus enthalten, der mindestens ein quaternäres Stickstoffatom aufweist.

Bevorzugte kationische direktziehenden Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die Verbindungen DZ1 bis DZ9, die im Prioritätsdokument:auf den Seiten 10 bis 12 offenbart werden. Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe. Als nichtionische direktziehende Farbstoffe eignen sich insbesondere nichtionische Nitro- und Chinonfarbstoffe und neutrale Azofarbstoffe. Geeignete blaue Nitrofarbstoffe sind insbesondere: 1,4-Bis[(2-hydroxyethyl)amino]-2-nitrobenzol, 1-(2-Hydroxyethyl)amino-2-nitro-4-[di(2-hydroxyethyl)amino]-benzol (HC Blue 2), 1-Methylamino-4- [methyl-(2,3-dihydroxypropyl)amino]-2-nitrobenzol (HC Blue 6),1-[(2,3- Dihydroxypropyl)-amino]-4-[ethyl-(2-hydroxyethyl)amino]-2-nitrobenzol-hydrochlorid (HC Blue 9), 1-[(2,3-Dihydroxypropyl)amino]-4-[methyl-(2-hydroxyethyl)amino]-2-nitrobenzol (HC Blue 10), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 2-((4-Amino-2-nitrophenyl)amino)-5-dimethylamino-benzoesäure (HC Blue 13), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2), 1-(2-Aminoethylamino)-4-[di(2-hydroxyethyl)amino]-2-nitrobenzol, 4-(Di(2-hydroxyethyl)amino)-2-nitro-1-phenylamino-benzol.

Geeignete rote Nitrofarbstoffe sind insbesondere: 1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)methylamino]-1-(methylamino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)-amino]-benzol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 4-(2,3-Dihydroxypropoxy)-1-[(2-hydroxyethyl)amino]-2-nitrobenzol (HC Orange 3), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3- Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, 7-Amino-3,4-dihydro-6-nitro-2H-1,4-benzoxazin (HC Red 14).

Geeignete gelbe Nitrofarbstoffe sind insbesondere: 1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[Di(2-hydroxyethyl)amino]-5-nitrophenol, 2-[(2- Hydroxyethyl)-amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)-amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13), 4-[(2-Hydroxyethyl)-amino]-3-nitro-benzonitril (HC Yellow 14), 4-[(2-Hydroxyethyl)amino]-3-nitro-benzamid (HC Yellow 15) 3-[(2-Hydroxyethyl)amino]-4-methyl-1-nitrobenzol, 4-Chlor-3-[(2-hydroxyethyl)amino]-1-nitrobenzol.

Geeignete Chinonfarbstoffe sind insbesondere: 1,4-Di[(2,3-dihydroxypropyl)amino]-9,10-anthrachinon, 1,4-Di[(2-hydroxyethyl)amino]-9,10-anthrachinon (C.I. 61,545, Disperse Blue 23), 1-[(2-Hydroxyethyl)amino]-4-methylamino-9,10-anthrachinon (C.I. 61,505, Disperse Blue 3), 2-[(2-Aminoethyl)amino]-9,10-anthrachinon (HC Orange 5), 1-Amino-4-hydroxy-9,10-anthrachinon (C.I. 60,710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (C.I. 75,470, Natural Red 4), 1-[(3-Aminopropyl)amino]-4-methylamino-9,10-anthrachinon (HC Blue 8), 1-[(3-Aminopropyl)-amino]-9,10- anthrachinon (HC Red 8), 1,4-Diamino-2-methoxy-9,10-anthrachinon (C.I. 62,015, Disperse Red 11, Solvent Violet No. 26), 1,4-Dihydroxy-5,8-bis[(2-hydroxyethyl)amino]-9,10-anthrachinon (C.I. 62,500, Disperse Blue 7, Solvent Blue No. 69), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-{6-[(3-Chlor-4-(methylamino)phenyl)imino]-4-methyl-3-oxo-1,4-cyclohexadien-1-yl}harnstoff (HC Red 9), 2-{{4-[Di(2-hydroxyethyl)amino]phenyl}amino}-5-[(2-hydroxyethyl)amino]-2,5-cyclohexadien-1,4-dion (HC Green 1), 5-Hydroxy-1,4-naphthochinon (C.I. 75,500, Natural Brown 7), 2-Hydroxy-1,4-naphthochinon (C.I. 75,480, Natural Orange 6), 1,2-Dihydro-2-(1,3-dihydro-3-oxo-2H-indol-2-yliden)-3H-indol-3-on (C.I. 73,000), 4-{{5-[(2-Hydroxyethyl) amino]-1-methyl-1H-pyrazol-4-yl}imino}-4,5-dihydro-5-[(2-hydroxyethyl)-imino]-1-methyl-1H-Pyrazol-sulfat(1:1), Hydrat(1:1).

Geeignete neutrale Azofarbstoffe sind insbesondere: 1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4- Aminophenyl)-azo]-1-[di(2-hydroxyethyl)amino]-3-methylbenzol (HC Yellow 7), 2,6-Diamino-3-[(pyridin-3-yl)azo]-pyridin, 2-{[4-(Acetylamino)phenyl]azo}-4-methylphenol (C.I. 11855; Disperse Yellow 3), 4-[(4-Nitrophenyl)azo]-anilin (C.I. 11,005; Disperse Orange 3).

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, daß die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Erfindungsgemäß besonders bevorzugte Haarbehandlungsmittel sind dadurch gekennzeichnet, dass die Aerosolzusammensetzung - bezogen auf ihr Gewicht - 0,025 bis 0,9 Gew.-%, vorzugsweise 0,03 bis 0,8 Gew.-%, weiter bevorzugt 0,04 bis 0,7 Gew.-%, noch weiter bevorzugt 0,05 bis 0,6 Gew.-%, besonders bevorzugt 0,06 bis 0,5 Gew.-% und insbesondere 0,07 bis 0,2 Gew.-% Farbstoff(e) aus der Gruppe
- 6-Hydroxy-5-[(4-sulfophenyl)azo]-2-naphthalinsulfonsäuredinatriumsalz (C.I. 15,985),
- 2,4-Dinitro-1-naphthol-7-sulfonsäure-dinatriumsalz (C.I. 10,316),
- 2-(Indan-1,3-dion-2-yl)chinolin-x,x-sulfonsäure (C.I. 47,005),
- 4-((4-Amino-3-sulfophenyl)azo)benzolsulfonsäure-dinatriumsalz (C.I. 13,015),
- 5-Hydroxy-1-(4-sulfophenyl)-4-[(4-sulfophenyl)azo]pyrazol-3-carbonsäure-trinatriumsalz (C.I. 19,140),
- 3-[(4-Phenylamino)phenyl]azobezolsulfonsäuresäure-natriumsalz (C.I. 13,065),
- 9-(2-Carboxyphenyl)-6-hydroxy-3H-xanthen-3-on (C.I. 45,350),
- 5-[(2,4-Dinitrophenyl)amino]-2-phenylaminobenzolsulfonsäure-natriumsalz (C.I. 10,385),
- 4-[(2,4- Dihydroxyphenyl)azo]-benzolsulfonsäure-natriumsalz (C.I. 14,270),
- 4-[(2-Hydroxynaphth-1-yl)azo]-benzolsulfonsäure-natriumsalz (C.I. 15,510),
- 4-[(2,4-Dihydroxy-3-[(2,4-dimethylphenyl)azo]-phenyl)azo]-benzolsulfonsäure-natriumsalz (C.I. 20,170),
- 4-Hydroxy-3-[(2-methoxyphenyl)azo]-1-naphthalinsulfonsäure-natriumsalz (C.I. 14,710;),
- 4-Hydroxy-3-[(4-sulfonaphth-1-yl)azo]-1-naphthalin-sulfonsäure-dinatriumsalz (C.I. 14,720),
- 6- Hydroxy-5-[(4-sulfonaphth-1-yl)azo]-2,4-naphthalin-disulfonsäure-trinatriumsalz (C.I. 16,255;),
- 3-Hydroxy-4-[(4-sulfonaphth-1-yl)azo]-2,7-naphthalin-disulfonsäure-trinatriumsalz (C.I. 16,185),
- 8-Amino-1-hydroxy-2-(phenylazo)-3,6-naphthalin-disulfonsäure-dinatriumsalz (C.I. 17,200),
- 5- (Acetylamino)-4-hydroxy-3-[(2-methylphenyl)azo]-2,7-naphthalin-disulfonsäure-dinatriumsalz (C.I. 18,065),
- 2-(3-Hydroxy-2,4,5,7-tetraiod-dibenzopyran-6-on-9-yl)-benzoesäure-dinatriumsalz (C.I. 45,430),
- N-[6-(Diethylamino)-9-(2,4-disulfophenyl)-3H-xanthen-3-yliden]-N-ethylethanammonium-hydroxid, inneres Salz, Natriumsalz (C.I. 45,100),
- 8-[(4-(Phenylazo)phenyl)azo]-7-naphthol-1,3-disulfonsäure-dinatriumsalz (C.I. 27,290),
- 2',4',5',7'-Tetrabrom-3',6'-dihydroxyspiro[isobenzofuran-1(3H),9'-[9H]xanthen]-3-on-dinatriumsalz (C.I. 45,380),
- 2',4',5',7'-Tetrabrom-4,5,6,7-tetrachlor-3',6'- dihydroxyspiro[isobenzofuran-1(3H),9'[9H]xanthen]-3-on-dinatriumsalz (C.I. 45,410),
- 3',6'-Dihydroxy-4',5'-diiodospiro[isobenzofuran-1(3H),9'(9H)- xanthen]-3-on-dinatriumsalz (C.I. 45425),
- 2-Hydroxy-3-((2- hydroxynaphth-1-yl)azo)-5-nitrobenzolsulfonsäure-natriumsalz (C.I. 15,685),
- 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red 195),
- 3-Hydroxy-4-[(4-methyl-2-sulfonphenyl)azo]-2-naphthalincarbonsäure-calciumsalz (C.I. 15,850:1),
- 3-[(2,4-Dimethyl-5-sulfophenyl)azo]-4-hydroxy-1-naphthalin-sulfonsäure-dinatriumsalz (C.I. 14,700),
- 1,4- Bis[(2-sulfo-4-methylphenyl)amino]-9,10-anthrachinon-dinatriumsalz (C.I. 61,570),
- Bis[4-(dimethylamino)phenyl]-(3,7-disulfo-2-hydroxynaphth-1-yl)carbenium-inneres Salz, Natriumsalz (C.I. 44,090),
- Bis[4-(diethylamino)-phenyl](2,4-disulfophenyl)carbenium-inneres Salz, Natriumsalz (2:1) (C.I. 42,045),
- Bis[4-(diethylamino)phenyl](5-hydroxy-2,4-disulfophenyl)-carbenium-inneres Salz, Calciumsalz (2:1) (C.I. 42,051),
- N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080),
- (2-Sulfophenyl)di[4-(ethyl((4-sulfophenyl)methyl)amino)phenyl]-carbenium-dinatriumsalz Betain (C.I. 42,090),
- 1-Amino-4-(phenylamino)-9,10-anthrachinon-2-sulfonsäure (C.I. 62,055),
- 1-Amino-4-(cyclohexylamino)-9,10-anthrachinon-2-sulfonsäure-natriumsalz (C.I. 62045),
- 2-(1,3-Dihydro-3-oxo-5-sulfo-2H-indol-2-yliden)-2,3-dihydro-3-oxo-1H-indol-5- sulfonsäure-dinatriumsalz (C.I. 73,015),
- 9-(2-Carboxyphenyl)-3-[(2-methylphenyl)amino]-6-[(2-methyl-4-sulfophenyl)amino]xanthylium-inneres Salz, Natriumsalz (C.I. 45,190),
- 1-Hydroxy-4-[(4-methyl-2- sulfophenyl)amino]-9,10-anthrachinon-natriumsalz (C.I. 60,730),
- Bis[3-nitro-4-[(4-phenylamino)-3-sulfo-phenylamino]-phenyl]-sulfon (C.I. 10,410),
- 5-Amino-4-hydroxy-6-[(4-nitrophenyl)-azo]-3-(phenylazo)-2,7-naphthalin-disulfonsäure-dinatriumsalz (C.I. 20,470),
- 3-Hydroxy-4-[(2-hydroxynaphth-1-yl)azo]-7-nitro-1-naphthalin-sulfonsäure-chromkomplex (3:2) (C.I. 15,711),
- 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440),
- 3',3",5',5"-Tetrabromphenolsulfonphthalein (Bromphenolblau), 3,4,5,6,3',3",5',5"-Octabromphenolsulfonphthalein (Tetrabromphenolblau),
- Mischungen der vorstehend genannten Farbstoffe,
enthält.

Zur verbesserten Trocknung enthalten die erfindungsmäßen Mittel als Trocknungsmittel - bezogen auf ihr Gewicht- 0,1 bis 10 Gew.-% Talkum.

Neben den zwingend erforderlichen Komponenten können die erfindungsgemäßen Haarbehandlungsmittel in der im Druckbehälter befindlichen Zusammensetzungen weitere Inhaltsstoffe enthalten, die den Mitteln und den mit Ihnen behandelten Haaren weitere Vorteile verleihen.

So kann die Aerosolzusammensetzung neben 20 bis 80 Gew.-% mindestens eines Treibmittels, 1 bis 79 Gew.-% mindestens eines Weißpigments; 0,1 bis 50 Gew.-% mindestens eines Viskositätsgebers und 0,01 bis 1 Gew.-% Farbstoff(en) bis zu 78,89 Gew.-% weitere Inhaltsstoffe enthalten. Es ist allerdings bevorzugt, dass mindestens 40 Gew.-%, weiter bevorzugt mindestens 45 Gew.-%, noch weiter bevorzugt mindestens 50 Gew.-%, besonders bevorzugt mindestens 55 Gew.-%, ganz besonders bevorzugt mindestens 60 Gew.-% und insbesondere mindestens 65 Gew.-% der im Druckbehälter enthaltenen Aerosolzusammensetzung aus den zwingenden Inhaltsstoffen a) bis d) gebildet werden.

Es hat sich gezeigt, dass die Haltbarkeit der Färbung auf der einen Seite und die Auswaschbarkeit auf der anderen Seite verbessert werden können, wenn Feuchthaltemittel in die Aerosolzusammensetzung inkorporiert werden.

Als Feuchthaltemittel eignen sich beispielsweise Sorbit bzw. Glycerin bzw. 1,2-Propylenglycol. Daneben eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)n-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Erfindungsgemäß besonders bevorzugte Haarbehandlungsmittel sind dadurch gekennzeichnet, daß die Aerosolzusammensetzung - bezogen auf ihr Gewicht - 0,1 bis 10 Gew.-%, vorzugsweise 0,5 bis 7,5 Gew.-%, weiter bevorzugt 0,75 bis 5 Gew.-%, noch weiter bevorzugt 1 bis 4 Gew.-%, besonders bevorzugt 1,1 bis 3 Gew.-% und insbesondere 1,2 bis 2,5 Gew.-% Feuchthaltemittel der Gruppe Propandiol, Glycerin, Polyethylenglycol(e), Butylenglycol, Sorbitol oder deren Mischungen enthält.

Insbesondere beim Einsatz löslicher Farbstoffe hat es sich bewährt, einwertige Alkohole, vorzugsweise Ethanol und/oder Isopropanol in die Aerosolzusammensetzung einzuarbeiten. Hier haben sich Mengen von 10 bis 40 Gew.-%, bezogen auf die treibmittelhaltige Zusammensetzung, als besonders geeignet erwiesen. Bevorzugte erfindungsgemäße Haarbehandlungsmittel sind dadurch gekennzeichnet, daß die Aerosolzusammensetzung - bezogen auf ihr Gewicht - 11 bis 39 Gew.-%, vorzugsweise 12 bis 38 Gew.-%, weiter bevorzugt 13 bis 37 Gew.-%, noch weiter bevorzugt 14 bis 36 Gew.-%, besonders bevorzugt 15 bis 35 Gew.-% und insbesondere 17,5 bis 32,5 Gew.-% Ethanol, Isopropanol öder deren Mischungen enthält.

In einigen Fällen ist ein mattiert-deckendes Aussehen der gefärbten Haare gewünscht, in anderen soll lediglich ein übermäßiger Glanz vermieden werden. Unabhängig davon soll die Zusammensetzung - insbesondere, wenn sie Wasser oder größere Mengen einwertiger Alkohole enthält - schnell auf den keratinischen Fasern trocknen. Es hat sich gezeigt, dass die Applizierbarkeit und Haltbarkeit der Färbung verbessert wird, wenn bestimmte Trocknungsmittel in die Aerosolzusammensetzung inkorporiert werden (siehe oben). Hier sind erfindungsgemäße Haarbehandlungsmittel bevorzugt, bei denen die Aerosolzusammensetzung - bezogen auf ihr Gewicht- 0,5 bis 7,5 Gew.-%, weiter bevorzugt 0,75 bis 5 Gew.-%, noch weiter bevorzugt 1 bis 4 Gew.-%, besonders bevorzugt 1,1 bis 3 Gew.-% und insbesondere 1,2 bis 2,5 Gew.-% Talkum, enthält.

Als weiteren Bestandteil können die erfindungsgemäßen Zusammensetzungen mindestens ein festigendes Polymer enthalten. Dieses kann die Haftung der Färbung auf dem Haar verbessern, der Frisur zusätzlich Halt geben und ein gleichmäßigeres Erscheinungsbild unterstützen.

Dabei können diese filmbildenden und/oder festigenden Polymere sowohl permanent als auch temporär kationisch, anionisch, nichtionisch oder amphoter sein. Weiterhin umfasst die vorliegende Erfindung auch die Erkenntnis, dass bei der Verwendung von mindestens zwei filmbildenden und/oder festigenden Polymeren diese selbstverständlich unterschiedliche Ladungen aufweisen können. Erfindungsgemäß bevorzugt kann es sein, wenn ein ionisches filmbildendes und/oder festigendes Polymer mit einem amphoteren und/oder nichtionischem filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere.

Dennoch sollen beispielhaft einige filmbildende Polymere beschrieben werden. Es wird an dieser Stelle jedoch explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C₁- bis C₇-Alkylgruppen, besonders bevorzugt C₁- bis C₃-Alkylgruppen sind.

Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/Polypropylenglykol-Copolymere, die beispielsweise, unter den Handelsbezeichnungen Ucon® der Union Carbide vertrieben werden.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI® von der Firma Lehmann & Voss, Hamburg, vertrieben wird. Festigende Polymere tragen zum Halt und/oder zum Aufbau des Haarvolumens und der Haarfülle der Gesamtfrisur bei. Diese sogenannten festigenden Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern.

Dadurch wird das schlaffe Herunterhängen der Haarsträhnen vermindert und somit ein langanhaltender Frisurenaufbau und -erhalt gewährleistet. Als Testmethode hierfür wird häufig der sogenannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste der erfindungsgemäß bevorzugt zu verwendenden Polymere finden sich somit selbstverständlich gerade auch die genannten filmbildenden Polymere wieder.

Beispiele für gebräuchliche filmbildende, festigende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate.

Eine besonders ausgeprägte Verbesserung des Frisurenhalts lässt sich erzielen, wenn ein filmbildendes und/oder festigendes Polymer eingesetzt wird, das spröde, harte Polymerfilme ausbildet. Vorzugsweise werden daher entsprechende filmbildende und/oder festigende Polymere verwendet.

Vorzugsweise enthält das erfindungsgemäße Mittel daher mindestens ein filmbildendes und/oder festigendes Polymer ausgewählt aus
- Aminomethylpropanolsalzen von Copolymeren des Allylmethacrylats mit einem oder mehreren Monomeren ausgewählt aus Acrylsäure, Methacrylsäure, Acrylsäureester und Methacrylsäureester,
- Vinylpyrrolidon-Vinylacetat-Copolymeren,
- Vinylpyrrolidon-Vinylcaprolactam-Dimethylaminopropylacrylamid-Copolymeren,
- Copolymeren des Octylacrylamids mit t-Butylaminoethylmethacrylat und zwei oder mehr Monomeren ausgewählt aus Acrylsäure, Methacrylsäure, Acrylsäureester und Methacrylsäureester, und
- Copolymeren der C₁₋₂-Alkylsuccinate mit Hydroxyalkylacrylaten und einem oder mehreren Monomeren ausgewählt aus Acrylsäure, Methacrylsäure, Acrylsäureester und Methacrylsäureester.

Entsprechende filmbildende und/oder festigende Polymere sind kommerziell erhältlich.

Besonders bevorzugt enthält das erfindungsgemäße Mittel als filmbildendes und/oder festigendes Polymer ein Aminomethylpropanolsalz eines Copolymers des Allylmethacrylats mit einem oder mehreren Monomeren, ausgewählt aus Acrylsäure, Methacrylsäure, Acrylsäureester und Methacrylsäureester.

Vorzugsweise handelt es sich bei den genannten Acrylsäureestern und Methacrylsäureestern um C₁-C₁₂-Alkylacrylate und C₁-C₁₂-Alkylmethacrylate, besonders bevorzugt um Methylacrylat, Ethylacrylat, Propylacrylat, Methylmethacrylat, Ethylmethacrylat, Propylmethacrylat und deren Mischungen.

Als Aminomethylpropanolsalz von Copolymeren des Allylmethacrylats mit einem oder mehreren Monomeren ausgewählt aus Acrylsäure, Methacrylsäure, Acrylsäureester und Methacrylsäureester, wird vorzugsweise das Copolymer mit der INCI-Bezeichnung AMP-Acrylates/Allyl Methacrylate Copolymer eingesetzt, das von der Firma Noveon unter der Bezeichnung Fixate™ G-100 vertrieben wird. Das erfindungsgemäße Mittel enthält dieses Copolymer ganz besonders bevorzugt.

Ein bevorzugtes Vinylpyrrolidon-Vinylacetat-Copolymer ist das PVP/VA Copolymer 60-40 W (INCI-Bezeichnung: VP/VA Copolymer, Aqua, Laurtrimonium Chloride).

Als Vinylpyrrolidon-Vinylcaprolactam/Dimethylaminopropylacrylamid-Copolymer wird vorzugsweise das von ISP unter der Bezeichnung Aquaflex SF 40 erhältliche Copolymer mit der INCI-Bezeichnung VP/Vinyl Caprolactam/DMAPA Acrylates Copolymer eingesetzt.

Ein bevorzugtes Copolymer des Octylacrylamids mit t-Butylaminoethylmethacrylat und zwei oder mehr Monomeren ausgewählt aus Acrylsäure, Methacrylsäure, Acrylsäureester und Methacrylsäureester, ist das von National Starch unter der Bezeichnung Amphomer® erhältliche Copolymer mit der INCI-Bezeichnung Octylacrylamide/Acrylates Butylaminoethyl Methacrylates Copolymer.

Als Copolymer der C₁₂-Atkytsuccinate mit Hydroxyalkylacrylaten und einem oder mehreren Monomeren ausgewählt aus Acrylsäure, Methacrylsäure, Acrylsäureester und Methacrylsäureester, ist das von ISP unter der Bezeichnung Allianz™ LT 120 erhältliche Copolymer mit der INCI-Bezeichnung Acrylates/C1-2 Succinates/Hydroxyacrylates Copolymer bevorzugt.

Erfindungsgemäß besonders bevorzugte Haarsprays sind dadurch gekennzeichnet, dass die Aerosolzusammensetzung - bezogen auf ihr Gewicht - festigende(s) Polymer(e) in einer Gesamtmenge von 0,2 Gew.-% bis 17,5 Gew.-%, vorzugsweise von 0,5 Gew.-% bis 15 Gew.-%, besonders bevorzugt von 2,0 Gew.-% bis 10,0 Gew.-% und insbesondere von 3,0 bis 5,0 Gew.-%, jeweils bezogen auf das Gewicht des Mittels, enthält.

Ganz besonders bevorzugte erfindungsgemäße Haarbehandlungsmittel sind dadurch gekennzeichnet, daß die Aerosolzusammensetzung - bezogen auf ihr Gewicht - 0,1 bis 10 Gew.-%, vorzugsweise 0,5 bis 7,5 Gew.-%, weiter bevorzugt 0,75 bis 5 Gew.-%, noch weiter bevorzugt 1 bis 4 Gew.-%, besonders bevorzugt 1,1 bis 3 Gew.-% und insbesondere 1,2 bis 2,5 Gew.-% festigende(s) Polymer(e) ausgewählt aus
- nichtionischen Polymeren auf Basis ethylenisch ungesättigter Monomere, insbesondere aus
- Homopolymeren des N-Vinylpyrrolidons,
- nichtionischen Copolymeren des N-Vinylpyrrolidons,
- Homopolymeren und nichtionischen Copolymeren des N-Vinylcaprolactams,
- Copolymeren des (Meth)acrylamids,
- Polyvinylalkohol, Polyvinylacetat,
- Chitosan und Derivaten des Chitosans,
- kationischen Cellulosederivaten,
- kationischen Copolymeren des 3-(C₁ bis C₆)-Alkyl-1-vinyl-imidazoliniums,
- Homopolymeren und Copolymeren enthaltend die Struktureinheit der Formel (M-1) 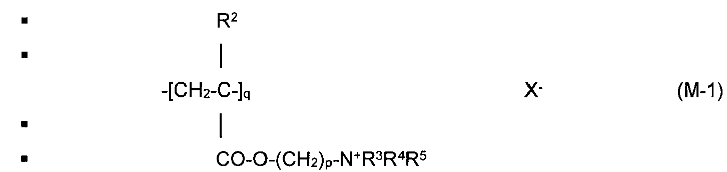 in der R²= -H oder -CH₃ ist, R³, R⁴ und R⁵ unabhängig voneinander ausgewählt sind aus (C₁ bis C₄)-Alkyl-, (C₁ bis C₄)-Alkenyl- oder (C₂ bis C₄)-Hydroxyalkylgruppen, p = 1, 2, 3 oder 4, q eine natürliche Zahl und X- ein physiologisch verträgliches organisches oder anorganisches Anion ist,
- anionischen Polymeren, welche Carboxylat- und/oder Sulfonatgruppen aufweisen,
- anionischen Polyurethanen
enthält.

Es hat sich gezeigt, daß ein bestimmtes Copolymer in der erfindungsgemäßen Kombination außerordentlich gute Haltegrade liefert, die durch den Einsatz der nachfolgend beschriebenen ester nicht nur verstärkt, sondern durch ein excellentes Haargefühl ergänzt werden. Erfindungsgemäß bevorzugte Haarsprays sind daher dadurch gekennzeichnet, daß die Aerosolzusammensetzung - bezogen auf ihr Gewicht - 0,1 bis 10 Gew.-%, vorzugsweise 0,25 bis 9 Gew.-%, weiter bevorzugt 0,5 bis 8 Gew.-%, besonders bevorzugt 0,75 bis 7 Gew.-% und insbesondere 1 bis 6 Gew.-% Copolymere(e) aus N-Octylacrylamid/Acrylsäure/tert.-Butylaminoethylmethacrylat (INCI-Bezeichnung: Octylacrylamide / Acrylates / Butylaminoethyl Methacrylate Copolymer) enthält.

Die erfindungsgemäßen Mittel enthalten vorzugsweise mindestens eine oberflächenaktive Substanz, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind. Diese Stoffe unterstützen die Mischung der einzelnen Komponenten der Aerosolzusammensetzung, erleichtern das Austreiben aus dem Druckbehälter und verbessern darüber hinaus das Sprühbild. Auch tragen sie zu einer stabilen Emulgierung von eventuel vorhandenen Parfümölen bei.

Erfindungsgemäß bevorzugte Haarbehandlungsmittel sind dadurch gekennzeichnet, dass die Aerosolzusammensetzung - bezogen auf ihr Gewicht - 0,05 bis 2,5 Gew.-%, vorzugsweise 0,1 bis 2 Gew.-% und insbesondere 0,25 bis 1 Gew.-% anionische(s) und/oder nichtionische(s) und/oder kationische(s) und/oder amphotere(s) Tensid(e), enthält.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethyl-ammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Weiter bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Besonders bevorzugte anionische Tenside sind die Alkali- oder Ammoniumsalze des Laurylethersulfates mit einem Ethoxylierungsgrad von 2 bis 4 EO.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethyl-ammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße Haarbehandlungsmittel sind dadurch gekennzeichnet, daß die Aerosolzusammensetzung amphotere(s) Tensid(e) aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Kokosalkylaminopropionat, Kokosacylaminoethylaminopropionat C₁₂-C₁₈ - Acylsarcosin, N-Alkyl-N,N-dimethylammonium-glycinate, beispielsweise Kokosalkyl-dimethylammoniumglycinat, N-Acyl-aminopropyl-N,N-dimethylammoniumglycinate, beispielsweise Kokosacylaminopropyl-dimethylammoniumglycinat, 2-Alkyl-3-carboxymethyl-3-hydroxyethyl-imidazoline mit jeweils 8 bis 18 C-Atomen in der Alkyl- oder Acylgruppe, Kokosacylaminoethylhydroxyethylcarboxymethylglycinat, der unter der INCI-Bezeichnung Cocamidopropyl Betain bekannten Verbindungen, der unter der INCI-Bezeichnung Disodium Cocoamphodiacetate bekannten Verbindungen enthalten, wobei bevorzugte Mittel das bzw. die amphotere(n) Tensid(e) in Mengen von 0,5 bis 9 Gew.-%, vorzugsweise von 0,75 bis 8 Gew.-% und insbesondere von 1 bis 7,5 Gew.-%, jeweils bezogen auf das gesamte Mittel, enthalten.

Erfindungsgemäß einsetzbar sind kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Besonders bevorzugte erfindungsgemäße Haarbehandlungsmittel sind dadurch gekennzeichnet, daß sie als kationischen Pflegestoff - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Tensid(e) aus der Gruppe der quartären Ammoniumverbindungen und/oder der Esterquats und/oder der Amidoamine enthalten, wobei bevorzugte kationische(s) Tensid(e) ausgewählt ist/sind aus Alkyltrimethylammoniumchloriden mit vorzugsweise 10 bis 18 Kohlenstoffatomen im Alkylrest und/oder Dialkyldimethylammoniumchloride mit vorzugsweise 10 bis 18 Kohlenstoffatomen im Alkylrest und/oder Trialkylmethylammoniumchloride mit vorzugsweise 10 bis 18 Kohlenstoffatomen im Alkylrest und/oder Cetyltrimethylammoniumchlorid und/oder Stearyltrimethylammoniumchlorid und/oder Distearyldimethylammoniumchlorid und/oder Lauryldimethylammoniumchlorid und/oder Lauryldimethylbenzylammoniumchlorid und/oder Tricetylmethylammoniumchlorid Quaternium-27 und/oder Quaternium-83 und/oder N-Methyl-N(2-hydroxyethyl)-N,N-(ditalgacyloxyethyl)ammonium-methosulfat und/oder N-Methyl-N(2-hydroxyethyl)-N,N-(distearoyloxyethyl)ammonium-methosulfat und/oder N,N-Dimethyl-N,N-distearoyloxyethyl-ammoniumchlorid und/oder N,N-Di-(2-hydroxyethyl)-N,N-(fettsäureesterethyl)-ammoniumchlorid.

Die erfindungsgemäßen Produkte können in einer weiteren Ausführungsform zusätzlich mindestens ein Silikon, insbesondere mindestens ein Silikonöl, enthalten. Erfindungsgemäß geeignete Silikonöle sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Unter dem Begriff Silikonöle versteht der Fachmann mehrere Strukturen Silicium-organischer Verbindungen. Zunächst werden hierunter die Dimethiconole verstanden.

Dimethicone bilden die zweite Gruppe der Silikone, welche erfindungsgemäß enthalten sein können. Diese können sowohl linear als auch verzweigt als auch cyclisch oder cyclisch und verzweigt sein. Dimethiconcopolyole bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning® 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohlbekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole besonders bevorzugt sein.

Die erfindungsgemäßen Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als weitere geeignete Hilfs- und Zusatzstoffe sind insbesondere Pflegestoffe zu nennen.

Als Pflegestoff enthalten die erfindungsgemäßen Mittel gegebenenfalls zusätzlich mindestens ein Pflanzenextrakt. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Geeignete Pflanzenextrakte werden durch Extraktion mit organischen Lösemitteln (wie beispielsweise Ethanol, Isopropanol, Diethylether, Benzin, Benzol, Chloroform) oder durch Wasserdampfdestillation gewonnen. Erfindungsgemäß sind vor allem die Extrakte aus Bambus, Leinsamen, Seerose, grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Der zusätzliche Pflanzenextrakt ist bevorzugt in dem erfindungsgemäßen Mittel in einer Menge von 0,05 Gew.-% bis 1,0 Gew.-%, insbesondere von 0,1 Gew.-% bis 0,5 Gew.-%, jeweils bezogen auf das Gewicht des kosmetischen Mittels, enthalten.

Weiterhin können auch kationisierte Proteinhydrolysate als Pflegekomponenten enthalten sein, wobei das zugrundeliegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann.

Als Pflegestoff kann weiterhin mindestens ein Vitamin, ein Provitamin, eine Vitaminvorstufe und/oder eines derer Derivate eingesetzt werden. Dabei sind erfindungsgemäß solche Vitamine, Pro-Vitamine und Vitaminvorstufen bevorzugt, die üblicherweise den Gruppen A, B, C, E, F und H zugeordnet werden. Besonders bevorzugt sind Vitamine, die zur B-Gruppe oder zu dem Vitamin B-Komplex gehören, ganz besonders bevorzugt Vitamin B₅ (Pantothensäure, Panthenol und Pantolacton). Weitere geeignete Pflegestoffe sind Proteinhydrolysate und/oder deren Derivate, wobei die Verwendung von Proteinhydrolysaten pflanzlichen Ursprungs, z. B. Soja-, Mandel-, Erbsen-, Kartoffel- und Weizenproteinhydrolysaten, bevorzugt ist. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Neben den Pflegestoffen können auch weitere Hilfs- und Zusatzstoffe zugegeben werden.

Durch Zugabe eines UV-Filters können sowohl die Zubereitungen selbst, als auch die behandelten Fasern vor schädlichen Einflüssen von UV-Strahlung geschützt werden. Es kann daher vorteilhaft sein, den erfindungsgemäßen kosmetischen Mitteln zusätzlich mindestens einen UV-Filter zuzugeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

### Beispiele:

Es wurden folgende erfindungsgemäße Aerosolzusammensetzungen hergestellt und in handelsüblichen Aluminiumdosen verpackt:

| | **weiß** | **gelb** | **pink** | **rot** | **blau** | **grün** |
|---|---|---|---|---|---|---|
| Butan | 24,5 | 24,5 | 24,5 | 24,5 | 24,5 | 24,5 |
| Propan | 24,0 | 24,0 | 24,0 | 24,0 | 24,0 | 24,0 |
| Isobutan | 1,0 | 1,0 | 1,0 | 1,0 | 1,0 | 1,0 |
| Pentan | 0,5 | 0,5 | 0,5 | 0,5 | 0,5 | 0,5 |
| Ethanol | 20,7 | 19,6 | 19,6 | 19,6 | 19,6 | 19,6 |
| t-Butyl Alkohol | 0,02 | 0,02 | 0,02 | 0,02 | 0,02 | 0,02 |
| Glycerin | 1,43 | 1,43 | 1,43 | 1,43 | 1,43 | 1,43 |
| Cl 77947 (Zinkoxid) | 10,0 | 10,0 | 10,0 | 10,0 | 10,0 | 10,0 |
| Cl 47005 (Yellow 10) | - | 0,08 | - | - | - | 0,08 |
| Cl 17200 (Red 33) | - | - | 0,08 | - | - | - |
| Cl 60730 (Ext. Violet 2) | - | - | 0,045 | - | 0,086 | - |
| Cl 16035 (Red 40) | - | - | - | 0,086 | - | - |
| Cl 14720 | - | - | - | 0,08 | - | - |
| Cl 42090 (Blue 1) | - | - | - | - | 0,08 | 0,043 |
| Cl 42053 (Green 3) | - | - | - | - | - | 0,08 |
| Methyl Methacrylate Crosspolymer | 5,0 | 5,0 | 5,0 | 5,0 | 5,0 | 5,0 |
| Aluminum Starch Octenylsuccinate | 4,65 | 4,65 | 4,65 | 4,65 | 4,65 | 4,65 |
| Talkum | 2,5 | 2,5 | 2,5 | 2,5 | 2,5 | 2,5 |
| VP/VA Copolymer | 1,75 | 1,75 | 1,75 | 1,75 | 1,75 | 1,75 |
| PEG-40 Hydrogenated Castor Oil | 0,4 | 0,4 | 0,4 | 0,4 | 0,4 | 0,4 |
| Parfum (Fragrance) | 0,08 | 0,08 | 0,08 | 0,08 | 0,08 | 0,08 |
| Wasser | ad 100 | | | | | |

Am Beispiel des blau färbenden Produktes wurde das erfindungsgemäße Produkt E mit einem Vergleichsprodukt V verglichen, welches keinen Viskositätsgeber enthielt:

| | **E** | **V** |
|---|---|---|
| Butan | 24,5 | 24,5 |
| Propan | 24,0 | 24,0 |
| Isobutan | 1,0 | 1,0 |
| Pentan | 0,5 | 0,5 |
| Ethanol | 19,6 | 34,9 |
| t-Butyl Alkohol | 0,02 | 0,04 |
| Glycerin | 1,43 | 1,43 |
| Cl 77947 (Zinkoxid) | 10,0 | 5,0 |
| Cl 60730 (Ext. Violet 2) | 0,086 | 0,086 |
| Cl 42090 (Blue 1) | 0,08 | 0,08 |
| Methyl Methacrylate Crosspolymer | 5,0 | - |
| Aluminum Starch Octenylsuccinate | 4,65 | - |
| Talkum | 2,5 | - |
| VP/VA Copolymer | 1,75 | 1,75 |
| PEG-40 Hydrogenated Castor Oil | 0,4 | 0,4 |
| Parfum (Fragrance) | 0,08 | 0,08 |
| Wasser | ad 100 | |

Die Zusammensetzung V neigte bei Lagerung zum Absetzen und bei der Applikation zur Verstopfung des Ventils. Darüber hinaus war die Färbung auf dem Haar weniger haftfähig, so daß unbeabsichtigtes Abfärben auf die Kleidung auftrat.

## Patentansprüche

1. Haarbehandlungsmittel, umfassend einen Druckbehälter und - darin befindlich - eine Aerosolzusammensetzung, die - bezogen auf ihr Gewicht -
a) 20 bis 80 Gew.-% mindestens eines Treibmittels;
b) 1 bis 79 Gew.-% mindestens eines Weißpigments;
c) 0,1 bis 50 Gew.-% mindestens eines Viskositätsgebers,
d) 0 bis 1 Gew.-% Farbstoff(e),
e) 0,1 bis 10 Gew.-% Talkum,
enthält.

2. Haarbehandlungsmittel nach Anspruch 1, **dadurch gekennzeichnet, dass** die Aerosolzusammensetzung - bezogen auf ihr Gewicht - 20 bis 80 Gew.-%, vorzugsweise 22,5 bis 77,5 Gew.-%, weiter bevorzugt 25 bis 75 Gew.-%, noch weiter bevorzugt 27,5 bis 72,5 Gew.-% und insbesondere 30 bis 70 Gew.-% Treibmittel oder Treibmittelgemisch, ausgewählt aus Dimethylether, HFO1234yf, HFO1234ze, Propan, Propen, n-Butan, iso-Butan, iso-Buten, n-Pentan, Penten, iso-Pentan und iso-Penten oder deren Mischungen, enthält.

3. Haarbehandlungsmittel nach einem der Ansprüche 1 oder 2, **dadurch gekennzeichnet, dass** die Aerosolzusammensetzung - bezogen auf ihr Gewicht -1,5 bis 75 Gew.-%, vorzugsweise 2 bis 50 Gew.-%, weiter bevorzugt 2,5 bis 40 Gew.-%, noch weiter bevorzugt 3 bis 30 Gew.-%, besonders bevorzugt 3,5 bis 25 Gew.-% und insbesondere 4 bis 15 Gew.-% Weißpigment(e) aus der Gruppe Titandioxid, Zinkoxid, Zinksulfid, Lithopone, Aluminiumstärkeoctenylsuccinat oder deren Mischungen, enthält.

4. Haarbehandlungsmittel nach einem der Ansprüche 1 bis 3, **dadurch gekennzeichnet, dass** die Aerosolzusammensetzung - bezogen auf ihr Gewicht - 0,25 bis 40 Gew.-%, vorzugsweise 0,5 bis 30 Gew.-%, weiter bevorzugt 0,75 bis 20 Gew.-%, noch weiter bevorzugt 1 bis 15 Gew.-%, besonders bevorzugt 1,25 bis 10 Gew.-% und insbesondere 1,5 bis 7,5 Gew.-% Viskositätsgeber aus der Gruppe der ggf. modifizierten quellfähigen Schichtsilikate, der verdickenden Acrylpolymere oder deren Mischungen, enthält.

5. Haarbehandlungsmittel nach Anspruch 4, **dadurch gekennzeichnet, dass** die Aerosolzusammensetzung - bezogen auf ihr Gewicht - 0,25 bis 40 Gew.-%, vorzugsweise 0,5 bis 30 Gew.-%, weiter bevorzugt 0,75 bis 20 Gew.-%, noch weiter bevorzugt 1 bis 15 Gew.-%, besonders bevorzugt 1,25 bis 10 Gew.-% und insbesondere 1,5 bis 7,5 Gew.-% Viskositätsgeber aus der Gruppe der ggf. modifizierten Smektite, vorzugsweise aus der Gruppe der ggf. modifizierten Montmoriilonite und/oder der ggf. modifizierten Hectorite enthält, wobei das Reaktionsprodukt von N,N-Dimethyl-N-octadecyl-1-Octadecanaminiumchlorid mit Hectorit (INCI-Bezeichnung: DISTEARDIMONIUM HECTORITE) besonders bevorzugt ist.

6. Haarbehandlungsmittel nach Anspruch 4 oder 5, **dadurch gekennzeichnet, dass** die Aerosolzusammensetzung - bezogen auf ihr Gewicht - 0,25 bis 40 Gew.-%, vorzugsweise 0,5 bis 30 Gew.-%, weiter bevorzugt 0,75 bis 20 Gew.-%, noch weiter bevorzugt 1 bis 15 Gew.-%, besonders bevorzugt 1,25 bis 10 Gew.-% und insbesondere 1,5 bis 7,5 Gew.-% Viskositätsgeber aus der Gruppe der verzweigten (Meth-)Acrylate (INCI-Bezeichnung ACRYLATE CROSSPOLYMER) enthält, wobei ein verzweigtes (Co-)Polymer von Methylmethacrylsäure (INCI-Bezeichnung: METHYL METHACRYLATE CROSSPOLYMER) besonders bevorzugt ist.

7. Haarbehandlungsmittel nach einem der Ansprüche 1 bis 6, **dadurch gekennzeichnet, dass** die Aerosolzusammensetzung - bezogen auf ihr Gewicht - 0,025 bis 0,9 Gew.-%, vorzugsweise 0,03 bis 0,8 Gew.-%, weiter bevorzugt 0,04 bis 0,7 Gew.-%, noch weiter bevorzugt 0,05 bis 0,6 Gew.-%, besonders bevorzugt 0,06 bis 0,5 Gew.-% und insbesondere 0,07 bis 0,2 Gew.-% Farbstoff(e) aus der Gruppe
- 6-Hydroxy-5-[(4-sulfophenyl)azo]-2-naphthalinsulfonsäuredinatriumsalz (C.I. 15,985),
- 2,4-Dinitro-1-naphthol-7-sulfonsäure-dinatriumsalz (C.I. 10,316),
- 2-(Indan-1,3-dion-2-yl)chinolin-x,x-sulfonsäure (C.I. 47,005),
- 4-((4-Amino-3-sulfophenyl)azo)benzolsulfonsäure-dinatriumsalz (C.I. 13,015),
- 5-Hydroxy-1-(4-sulfophenyl)-4-[(4-sulfophenyl)azo]pyrazol-3-carbonsäure-trinatriumsalz (C.I. 19,140),
- 3-[(4-Phenylamino)phenyl]azobezolsulfonsäuresäure-natriumsalz (C.I. 13,065),
- 9-(2-Carboxyphenyl)-6-hydroxy-3H-xanthen-3-on (C.I. 45,350),
- 5-[(2,4-Dinitrophenyl)amino]-2-phenylaminobenzolsulfonsäure-natriumsalz (C.I. 10,385),
- 4-[(2,4- Dihydroxyphenyl)azo]-benzolsulfonsäure-natriumsalz (C.I. 14,270),
- 4-[(2-Hydroxynaphth-1-yl)azo]-benzolsulfonsäure-natriumsalz (C.I. 15,510),
- 4-[(2,4-Dihydroxy-3-[(2,4-dimethylphenyl)azo]-phenyl)azo]-benzolsulfonsäure-natriumsalz (C.I. 20,170),
- 4-Hydroxy-3-[(2-methoxyphenyl)azo]-1-naphthalinsulfonsäure-natriumsalz (C.I. 14,710;),
- 4-Hydroxy-3-[(4-sulfonaphth-1-yl)azo]-1-naphthalin-sulfonsäure-dinatriumsalz (C.I. 14,720),
- 6- Hydroxy-5-[(4-sulfonaphth-1-yl)azo]-2,4-naphthalin-disulfonsäure-trinatriumsalz (C.I. 16,255;),
- 3-Hydroxy-4-[(4-sulfonaphth-1-yl)azo]-2,7-naphthalin-disulfonsäure-trinatriumsalz (C.I. 16,185),
- 8-Amino-1-hydroxy-2-(phenylazo)-3,6-naphthalin-disulfonsäure-dinatriumsalz (C.I. 17,200),
- 5-(Acetylamino)-4-hydroxy-3-[(2-methylphenyl)azo]-2,7-naphthalin-disulfonsäure-dinatriumsalz (C.I. 18,065),
- 2-(3-Hydroxy-2,4,5,7-tetraiod-dibenzopyran-6-on-9-yl)-benzoesäure-dinatriumsalz (C.I. 45,430),
- N-[6-(Diethylamino)-9-(2,4-disulfophenyl)-3H-xanthen-3-yliden]-N-ethylethanammonium-hydroxid, inneres Salz, Natriumsalz (C.I. 45,100),
- 8-[(4-(Phenylazo)phenyl)azo]-7-naphthol-1,3-disulfonsäure-dinatriumsalz (C.I. 27,290),
- 2',4',5',7'-Tetrabrom-3',6'-dihydroxyspiro[isobenzofuran-1(3H),9'-[9H]xanthen]-3-on-dinatriumsalz (C.I. 45,380),
- 2',4',5',7'-Tetrabrom-4,5,6,7-tetrachlor-3',6'-dihydroxyspiro[isobenzofuran-1(3H),9'[9H]xanthen]-3-on-dinatriumsalz (C.I. 45,410),
- 3',6'-Dihydroxy-4',5'-diiodospiro[isobenzofuran-1(3H),9'(9H)- xanthen]-3-on-dinatriumsalz (C.I. 45425),
- 2-Hydroxy-3-((2- hydroxynaphth-1-yl)azo)-5-nitrobenzolsulfonsäure-natriumsalz (C.I. 15,685),
- 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red 195),
- 3-Hydroxy-4-[(4-methyl-2-sulfonphenyl)azo]-2-naphthalincarbonsäure-calciumsalz (C.I. 15,850:1),
- 3-[(2,4-Dimethyl-5-sulfophenyl)azo]-4-hydroxy-1-naphthalin-sulfonsäure-dinatriumsalz (C.I. 14,700),
- 1,4- Bis[(2-sulfo-4-methylphenyl)amino]-9,10-anthrachinon-dinatriumsalz (C.I. 61,570),
- Bis[4-(dimethylamino)phenyl]-(3,7-disulfo-2-hydroxynaphth-1-yl)carbenium-inneres Salz, Natriumsalz (C.I. 44,090),
- Bis[4-(diethylamino)-phenyl](2,4-disulfophenyl)carbenium-inneres Salz, Natriumsalz (2:1) (C.I. 42,045),
- Bis[4-(diethylamino)phenyl](5-hydroxy-2,4-disulfophenyl)-carbenium-inneres Salz, Calciumsalz (2:1) (C.I. 42,051),
- N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080),
- (2-Sulfophenyl)di[4-(ethyl((4-sulfophenyl)methyl)amino)phenyl]-carbenium-dinatriumsalz Betain (C.I. 42,090),
- 1-Amino-4-(phenylamino)-9,10-anthrachinon-2-sulfonsäure (C.I. 62,055),
- 1-Amino-4-(cyclohexylamino)-9,10-anthrachinon-2-sulfonsäure-natriumsalz (C.I. 62045),
- 2-(1,3-Dihydro-3-oxo-5-sulfo-2H-indol-2-yliden)-2,3-dihydro-3-oxo-1H-indol-5- sulfonsäure-dinatriumsalz (C.I. 73,015),
- 9-(2-Carboxyphenyl)-3-[(2-methylphenyl)amino]-6-[(2-methyl-4-sulfophenyl)amino]xanthylium-inneres Salz, Natriumsalz (C.I. 45,190),
- 1-Hydroxy-4-[(4-methyl-2-sulfophenyl)amino]-9,10-anthrachinon-natriumsalz (C.I. 60,730),
- Bis[3-nitro-4-[(4-phenylamino)-3-sulfo-phenylamino]-phenyl]-sulfon (C.I. 10,410),
- 5-Amino-4-hydroxy-6-[(4-nitrophenyl)-azo]-3-(phenylazo)-2,7-naphthalin-disulfonsäure-dinatriumsalz (C.I. 20,470),
- 3-Hydroxy-4-[(2-hydroxynaphth-1-yl)azo]-7-nitro-1-naphthalin-sulfonsäure-chromkomplex (3:2) (C.I. 15,711),
- 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440),
- 3',3",5',5"-Tetrabromphenolsulfonphthalein (Bromphenolblau), 3,4,5,6,3',3",5',5"-Octabromphenolsulfonphthalein (Tetrabromphenolblau),
- Mischungen der vorstehend genannten Farbstoffe,
enthält.

8. Haarbehandlungsmittel nach einem der Ansprüche 1 bis 7, **dadurch gekennzeichnet, daß** die Aerosolzusammensetzung - bezogen auf ihr Gewicht - 0,1 bis 10 Gew.-%, vorzugsweise 0,5 bis 7,5 Gew.-%, weiter bevorzugt 0,75 bis 5 Gew.-%, noch weiter bevorzugt 1 bis 4 Gew.-%, besonders bevorzugt 1,1 bis 3 Gew.-% und insbesondere 1,2 bis 2,5 Gew.-% Feuchthaltemittel der Gruppe Propandiol, Glycerin, Polyethylenglycol(e), Butylenglycol, Sorbitol oder deren Mischungen enthält.

9. Haarbehandlungsmittel nach einem der Ansprüche 1 bis 8, **dadurch gekennzeichnet, dass** die Aerosolzusammensetzung - bezogen auf ihr Gewicht - 0,5 bis 7,5 Gew.-%, weiter bevorzugt 0,75 bis 5 Gew.-%, noch weiter bevorzugt 1 bis 4 Gew.-%, besonders bevorzugt 1,1 bis 3 Gew.-% und insbesondere 1,2 bis 2,5 Gew.-% Talkum, enthält.

10. Haarbehandlungsmittel nach einem der Ansprüche 1 bis 8, **dadurch gekennzeichnet, dass** die Aerosolzusammensetzung - bezogen auf ihr Gewicht - 0,1 bis 10 Gew.-%, vorzugsweise 0,5 bis 7,5 Gew.-%, weiter bevorzugt 0,75 bis 5 Gew.-%, noch weiter bevorzugt 1 bis 4 Gew.-%, besonders bevorzugt 1,1 bis 3 Gew.-% und insbesondere 1,2 bis 2,5 Gew.-% festigende(s) Polymer(e) ausgewählt aus
- nichtionischen Polymeren auf Basis ethylenisch ungesättigter Monomere, insbesondere aus
- Homopolymeren des N-Vinylpyrrolidons,
- nichtionischen Copolymeren des N-Vinylpyrrolidons,
- Homopolymeren und nichtionischen Copolymeren des N-Vinylcaprolactams,
- Copolymeren des (Meth)acrylamids,
- Polyvinylalkohol, Polyvinylacetat,
- Chitosan und Derivaten des Chitosans,
- kationischen Cellulosederivaten,
- kationischen Copolymeren des 3-(C₁ bis C₆)-Alkyl-1-vinyl-imidazoliniums,
- Homopolymeren und Copolymeren enthaltend die Struktureinheit der Formel (M-1) 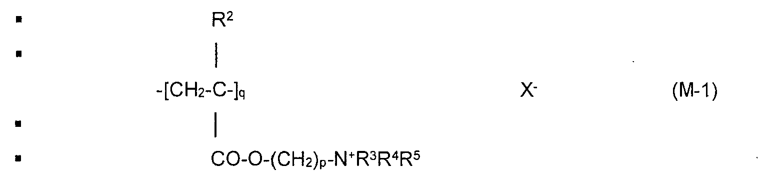 in der R²= -H oder -CH₃ ist, R³, R⁴ und R⁵ unabhängig voneinander ausgewählt sind aus (C₁ bis C₄)-Alkyl-, (C₁ bis C₄)-Alkenyl- oder (C₂ bis C₄)-Hydroxyalkylgruppen, p = 1, 2, 3 oder 4, q eine natürliche Zahl und X⁻ ein physiologisch verträgliches organisches oder anorganisches Anion ist,
- anionischen Polymeren, welche Carboxylat- und/oder Sulfonatgruppen aufweisen,
- anionischen Polyurethanen
enthält.

## Claims

1. A hair treatment agent comprising a pressurized container and an aerosol composition located therein which contains, based on the weight thereof,
a) 20 to 80 wt.% of at least one propellant,
b) 1 to 79 wt.% of at least one white pigment,
c) 0.1 to 50 wt.% of at least one viscosity enhancer,
d) 0 to 1 wt.% dye(s),
e) 0.1 to 10 wt.% talc.

2. The hair treatment agent according to claim 1, **characterized in that** the aerosol composition contains, based on the weight thereof, 20 to 80 wt.%, preferably 22.5 to 77.5 wt.%, more preferably 25 to 75 wt.%, even more preferably 27.5 to 72.5 wt.%, and in particular 30 to 70 wt.%, of propellant or propellant mixture selected from dimethyl ether, HFO1234yf, HFO1234ze, propane, propene, n-butane, iso-butane, isobutene, n-pentane, pentene, iso-pentane and iso-pentene, or mixtures thereof.

3. The hair treatment agent according to one of claims 1 or 2, **characterized in that** the aerosol composition contains, based on the weight thereof, 1.5 to 75 wt.%, preferably 2 to 50 wt.%, more preferably 2.5 to 40 wt.%, even more preferably 3 to 30 wt.%, particularly preferably 3.5 to 25 wt.%, and in particular 4 to 15 wt.%, of white pigment(s) from the group of titanium dioxide, zinc oxide, zinc sulfide, lithopone, aluminum starch octenylsuccinate or mixtures thereof.

4. The hair treatment agent according to one of claims 1 to 3, **characterized in that** the aerosol composition contains, based on the weight thereof, 0.25 to 40 wt.%, preferably 0.5 to 30 wt.%, more preferably 0.75 to 20 wt.%, even more preferably 1 to 15 wt.%, particularly preferably 1.25 to 10 wt.%, and in particular 1.5 to 7.5 wt.%, of viscosity enhancer from the group of optionally modified swellable phyllosilicates, thickening acrylic polymers or mixtures thereof.

5. The hair treatment agent according to claim 4, **characterized in that** the aerosol composition contains, based on the weight thereof, 0.25 to 40 wt.%, preferably 0.5 to 30 wt.%, more preferably 0.75 to 20 wt.%, even more preferably 1 to 15 wt.%, particularly preferably 1.25 to 10 wt.%, and in particular 1.5 to 7.5 wt.%, of viscosity enhancer from the group of optionally modified smectites, preferably from the group of optionally modified montmorillonites and/or of optionally modified hectorites, the reaction product of N,N-dimethyl-N-octadecyl-1-octadecanaminium chloride with hectorite (INCI name: DISTEARDIMONIUM HECTORITE) being particularly preferred.

6. The hair treatment agent according to claim 4 or 5, **characterized in that** the aerosol composition contains, based on the weight thereof, 0.25 to 40 wt.%, preferably 0.5 to 30 wt.%, more preferably 0.75 to 20 wt.%, even more preferably 1 to 15 wt.%, particularly preferably 1.25 to 10 wt.%, and in particular 1.5 to 7.5 wt.%, of viscosity enhancer from the group of branched (meth)acrylates (INCI name ACRYLATE CROSSPOLYMER), a branched (co)polymer of methyl methacrylic acid (INCI name: METHYL METHACRYLATE CROSSPOLYMER) being particularly preferred.

7. The hair treatment agent according to one of claims 1 to 6, **characterized in that** the aerosol composition contains, based on the weight thereof, 0.025 to 0.9 wt.%, preferably 0.03 to 0.8 wt.%, more preferably 0.04 to 0.7 wt.%, even more preferably 0.05 to 0.6% wt.%, particularly preferably 0.06 to 0.5% wt.%, and in particular 0.07 to 0.2 wt.%, of dye(s) from the group
- 6-hydroxy-5-[(4-sulfophenyl)azo]-2-naphthalenesulfonic acid disodium salt (C.I. 15985),
- 2,4-dinitro-1-naphthol-7-sulfonic acid disodium salt (C.I. 10316),
- 2-(indan-1,3-dione-2-yl)quinoline-x,x-sulfonic acid (C.I. 47005),
- 4-((4-amino-3-sulfophenyl)azo)benzenesulfonic acid disodium salt (C.I. 13015),
- 5-hydroxy-1-(4-sulfophenyl)-4-[(4-sulfophenyl)azo]pyrazole-3-carboxylic acid trisodium salt (C.I. 19140),
- 3-[(4-phenylamino)phenyl]azobenzenesulfonic acid sodium salt (C.I. 13065),
- 9-(2-carboxyphenyl)-6-hydroxy-3H-xanthen-3-one (C.I. 45350),
- 5-[(2,4-dinitrophenyl)amino]-2-phenylaminobenzenesulfonic acid sodium salt (C.I. 10385),
- 4-[(2,4-dihydroxyphenyl)azo]-benzenesulfonic acid sodium salt (C.I. 14270),
- 4-[(2-hydroxynaphth-1-yl)azo]-benzenesulfonic acid sodium salt (C.I. 15510),
- 4-[(2,4-dihydroxy-3-[(2,4-dimethylphenyl)azo]-phenyl)azo]-benzenesulfonic acid sodium salt (C.I. 20170),
- 4-hydroxy-3-[(2-methoxyphenyl)azo]-1-naphthalenesulfonic acid sodium salt (C.I. 14710),
- 4-hydroxy-3-[(4-sulfonaphth-1-yl)azo]-1-naphthalenesulfonic acid disodium salt (C.I. 14720),
- 6-hydroxy-5-[(4-sulfonaphth-1-yl)azo]-2,4-naphthalenedisulfonic acid trisodium salt (C.I. 16255),
- 3-hydroxy-4-[(4-sulfonaphth-1-yl)azo]-2,7-naphthalenedisulfonic acid trisodium salt (C.I. 16185),
- 8-amino-1-hydroxy-2-(phenylazo)-3,6-naphthalenedisulfonic acid disodium salt (C.I. 17200),
- 5-(acetylamino)-4-hydroxy-3-[(2-methylphenyl)azo]-2,7-naphthalenedisulfonic acid disodium salt (C.I. 18065),
- 2-(3-hydroxy-2,4,5,7-tetraiodo-dibenzopyran-6-on-9-yl)-benzoic acid disodium salt (C.I. 45430),
- N-[6-(diethylamino)-9-(2,4-disulfophenyl)-3H-xanthen-3-ylidene]-N-ethylethanammonium hydroxide, inner salt, sodium salt (C.I. 45100),
- 8-[(4-(phenylazo)phenyl)azo]-7-naphthol-1,3-disulfonic acid disodium salt (C.I. 27290),
- 2',4',5',7'-tetrabromo-3',6'-dihydroxyspiro[isobenzofuran-1(3H),9'-[9H]xanthene]-3-one disodium salt (C.I. 45380),
- 2',4',5',7'-tetrabromo-4,5,6,7-tetrachloro-3',6'-dihydroxyspiro[isobenzofuran-1 (3H),9'[9H]xanthene]-3-one disodium salt (C.I. 45410),
- 3',6'-dihydroxy-4',5'-diiodospiro[isobenzofuran-1(3H),9'(9H)-xanthene]-3-one disodium salt (C.I. 45425),
- 2-hydroxy-3-((2-hydroxynaphth-1-yl)azo)-5-nitrobenzenesulfonic acid sodium salt (C.I. 15685),
- 3-hydroxy-4-(3-methyl-5-oxo-1 -phenyl-4,5-dihydro-1H-pyrazol-4-ylazo)-naphthalene-1-sulfonic acid sodium salt, chromium complex (Acid Red 195),
- 3-hydroxy-4-[(4-methyl-2-sulfonphenyl)azo]-2-naphthalenecarboxylic acid calcium salt (C.I. 15850:1),
- 3-[(2,4-dimethyl-5-sulfophenyl)azo]-4-hydroxy-1-naphthalenesulfonic acid disodium salt (C.I. 14700),
- 1,4-bis[(2-sulfo-4-methylphenyl)amino]-9,10-anthraquinone disodium salt (C.I. 61570),
- bis[4-(dimethylamino)phenyl]-(3,7-disulfo-2-hydroxynaphth-1-yl)carbenium inner salt, sodium salt (C.I. 44090),
- bis[4-(diethylamino)-phenyl](2,4-disulfophenyl)carbenium inner salt, sodium salt (2:1) (C.I. 42045),
- bis[4-(diethylamino)phenyl](5-hydroxy-2,4-disulfophenyl)-carbenium inner salt, calcium salt (2:1) (C.I. 42051),
- N-[4-[(2,4-disulfophenyl)[4-[ethyl(phenylmethyl)amino)phenyl]methylene]-2,5-cyclohexadiene-1-ylidene]-N-ethylbenzene methanaminium hydroxide, inner salt, sodium salt (C.I. 42080),
- (2-sulfophenyl)di[4-(ethyl((4-sulfophenyl)methyl)amino)phenyl]-carbenium disodium salt betaine (C.I. 42090),
- 1-amino-4-(phenylamino)-9,10-anthraquinone-2-sulfonic acid (C.I. 62055),
- 1-amino-4-(cyclohexylamino)-9,10-anthraquinone-2-sulfonic acid sodium salt (C.I. 62045),
- 2-(1,3-dihydro-3-oxo-5-sulfo-2H-indol-2-ylidene)-2,3-dihydro-3-oxo-1H-indol-5-sulfonic acid disodium salt (C.I. 73015),
- 9-(2-carboxyphenyl)-3-[(2-methylphenyl)amino]-6-[(2-methyl-4-sulfophenyl)amino]xanthylium inner salt, sodium salt (C.I. 45190),
- 1-hydroxy-4-[(4-methyl-2-sulfophenyl)amino]-9,10-anthraquinone sodium salt (C.I. 60730),
- bis[3-nitro-4-[(4-phenylamino)-3-sulfophenylamino]-phenyl]-sulfone (C.I. 10410),
- 5-amino-4-hydroxy-6-[(4-nitrophenyl)-azo]-3-(phenylazo)-2,7-naphthalenedisulfonic acid disodium salt (C.I. 20470),
- 3-hydroxy-4-[(2-hydroxynaphth-1-yl)azo]-7-nitro-1-naphthalenesulfonic acid chromium complex (3:2) (C.I. 15711),
- 4-(acetylamino)-5-hydroxy-6-[(7-sulfo-4-[(4-sulfophenyl)azo]naphth-1-yl)azo]-1,7-naphthalenedisulfonic acid tetrasodium salt (C.I. 28440),
- 3',3",5',5"-tetrabromophenolsulfonephthalein (bromophenol blue), 3,4,5,6,3',3",5',5"-octabromophenolsulfonephthalein (tetrabromophenol blue),
- and mixtures of the above-mentioned dyes.

8. The hair treatment agent according to one of claims 1 to 7, **characterized in that** the aerosol composition contains, based on the weight thereof, 0.1 to 10 wt.%, preferably 0.5 to 7.5 wt.%, more preferably 0.75 to 5 wt.%, even more preferably 1 to 4 wt.%, particularly preferably 1.1 to 3 wt.%, and in particular 1.2 to 2.5 wt.%, of humectants from the group of propanediol, glycerol, polyethylene glycol(s), butylene glycol, sorbitol, or mixtures thereof.

9. The hair treatment agent according to one of claims 1 to 8, **characterized in that** the aerosol composition contains, based on the weight thereof, 0.5 to 7.5 wt.%, more preferably 0.75 to 5 wt.%, even more preferably 1 to 4 wt.%, particularly preferably 1.1 to 3 wt.%, and in particular 1.2 to 2.5 wt.%, of talc.

10. The hair treatment agent according to one of claims 1 to 8, **characterized in that** the aerosol composition contains, based on the weight thereof, 0.1 to 10 wt.%, preferably 0.5 to 7.5 wt.%, more preferably 0.75 to 5 wt.%, even more preferably 1 to 4 wt.%, particularly preferably 1.1 to 3 wt.%, and in particular 1.2 to 2.5 wt.%, of setting polymer(s) selected from
- non-ionic polymers based on ethylenically unsaturated monomers, in particular from
- homopolymers of N-vinylpyrrolidone,
- nonionic copolymers of N-vinylpyrrolidone,
- homopolymers and non-ionic copolymers of N-vinylcaprolactam,
- copolymers of (meth)acrylamide,
- polyvinyl alcohol, polyvinyl acetate,
- chitosan and chitosan derivatives,
- cationic cellulose derivatives,
- cationic copolymers of 3-(C₁ to C₆)-alkyl-1-vinyl-imidazolinium,
- homopolymers and copolymers containing the structural unit of formula (M-1) 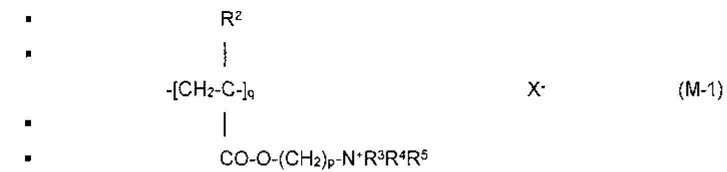 in which R² = -H or -CH₃; R³, R⁴ and R⁵ are selected, independently of one another, from (C₁ to C₄)-alkyl, (C₁ to C₄)-alkenyl or (C₂ to C₄)-hydroxyalkyl groups; p=1, 2, 3 or 4; q is a natural number and X⁻ is a physiologically acceptable organic or anorganic anion,
- anionic polymers which comprise carboxylate and/or sulfonate groups, and
- anionic polyurethanes.

## Revendications

1. Agent de traitement capillaire comprenant un récipient sous pression et, situé à l'intérieur de celui-ci, une composition aérosol contenant, par rapport à son poids,
a) de 20 à 80 % en poids d'au moins un agent de propulsion ;
b) de 1 à 79 % en poids d'au moins un pigment blanc ;
c) de 0,1 à 50 % en poids d'au moins un agent de viscosité,
d) de 0 à 1 % en poids de colorant(s),
e) de 0,1 à 10 % en poids de talc.

2. Agent de traitement capillaire selon la revendication 1, **caractérisé en ce que** la composition aérosol contient, par rapport à son poids, de 20 à 80 % en poids, de préférence de 22,5 à 77,5 % en poids, de manière préférée de 25 à 75 % en poids, de manière plus préférée de 27,5 à 72,5 % en poids, et en particulier de 30 à 70 % en poids d'agent de propulsion ou de mélange d'agents de propulsion choisi parmi l'éther diméthylique, le HFO1234yf, le HFO1234ze, le propane, le propène, le n-butane, l'iso-butane, l'iso-butène, le n-pentane, le pentène, l'iso-pentane et l'iso-pentène ou leurs mélanges.

3. Agent de traitement capillaire selon l'une quelconque des revendications 1 ou 2, **caractérisée en ce que** la composition aérosol contient, par rapport à son poids, de 1,5 à 75 % en poids, de préférence de 2 à 50 % en poids, de manière préférée de 2,5 à 40 % en poids, de manière plus préférée de 3 à 30 % en poids, de manière encore plus préférée de 3,5 à 25 % en poids et en particulier de 4 à 15 % en poids de pigment(s) blanc(s) du groupe dioxyde de titane, oxyde de zinc, sulfure de zinc, lithopone, octényle succinate d'aluminium amidon ou leurs mélanges.

4. Agent de traitement capillaire selon l'une quelconque des revendications 1 à 3, **caractérisé en ce que** la composition aérosol contient, par rapport à son poids, de 0,25 à 40 % en poids, de préférence de 0,5 à 30 % en poids, de manière préférée de 0,75 à 20 % en poids, de manière plus préférée de 1 à 15 % en poids, de manière encore plus préférée de 1,25 à 10 % en poids et en particulier de 1,5 à 7,5 % en poids d' agent de viscosité du groupe des silicates à couche gonflable éventuellement modifiés, des polymères acryliques épaississants ou leurs mélanges.

5. Agent de traitement capillaire selon la revendication 4, **caractérisé en ce que** la composition aérosol contient, par rapport à son poids, de 0,25 à 40 % en poids, de préférence de 0,5 à 30 % en poids, de manière préférée de 0,75 à 20 % en poids, de manière plus préférée de 1,25 à 10 % en poids et en particulier de 1,5 à 7,5 % en poids d'agent de viscosité, du groupe de smectite éventuellement modifiée, de préférence du groupe de montmorilonite éventuellement modifiée et/ou des hectoites éventuellement modifiées, dans lequel le produit de réaction du chlorure de N,N-diméthyl-N-octadécyl-1-octadécanaminium avec l'hectorite (désignation INCI: DISTEARDIMONIUM HECTORITE) est particulièrement préféré.

6. Agent de traitement capillaire selon la revendication 4 ou 5, **caractérisé en ce que** la composition aérosol contient, par rapport à son poids, de 0,25 à 40 % en poids, de préférence de 0,5 à 30 % en poids, de manière préférée de 0,75 à 20 % en poids, de manière plus préférée de 1 à 15 % en poids, de manière encore plus préférée de 1,25 à 10 % en poids et en particulier de 1,5 à 7,5 % en poids d'agent de viscosité du groupe des (méth)acrylates ramifiés (désignation INCI: ACRYLATE CROSSPOLYMER), dans lequel un (co)polymère ramifié d'acide méthylméthacrylique (désignation INCI : METHYL METHACRYLATE CROSSPOLYMER) est particulièrement préféré.

7. Agent de traitement capillaire selon l'une quelconque des revendications 1 à 6, **caractérisé en ce que** la composition aérosol contient, par rapport à son poids, de 0,025 à 0,9 % en poids, de préférence de 0,03 à 0,8 % en poids, de manière préférée de 0,04 à 0,7 % en poids, de manière plus préférée de 0,05 à 0,6 % en poids, de manière encore plus préférée de 0,06 à 0,5 % en poids et en particulier de 0,07 à 0,2 % en poids du (des) colorant(s) du groupe constitué du
- sel disodique de l'acide 6-hydroxy-5-[(4-sulfophényl)azo]-2-naphtalènesulfonique (C.I. 15,985),
- sel disodique de l'acide 2,4-dinitro-1-naphtol-7-sulfonique (C.I. 10,316),
- acide 2-(indan-1,3-dion-2-yl)quinoléine-x, x-sulfonique (C.I. 47,005),
- sel disodique de l'acide 4-((4-amino-3-sulfophényl)azo)benzènesulfonique (C.I. 13,015),
- sel trisodique de l'acide 5-hydroxy-1-(4-sulfophényl)-4-[(4-sulfophényl)azo]pyrazole-3-carboxylique (C.I. 19,140),
- sel sodique de l'acide 3-[(4-phénylamino)phényl]azobézènesulfonique (C.I. 13,065), 9-(2-carboxyphényl)-6-hydroxy-3H-xanthène-3-one (C.I. 45,350),
- sel sodique de l'acide 5-[(2,4-dinitrophényl)amino]-2-phénylaminobenzènesulfonique (C.I. 10,385),
- sel sodique de l'acide 4-[(2,4-dihydroxyphényl)azo]-benzènesulfonique (C.I. 14,270),
- sel sodique de l'acide 4-[(2- hydroxynapht -1-yl)azo]-benzènesulfonique (C.I. 15,510),
- sel sodique de l'acide 4-[(2,4-dihydroxy-3-[(2,4-diméthylphényl)azo]-phényl)azo]-benzènesulfonique (C.I. 20,170),
- sel sodique de l'acide 4-hydroxy-3-[(2-méthoxyphényl)azo]-1-naphtalènesulfonique (C.I. 14,710),
- sel disodique de l'acide 4-hydroxy-3-[(4-sulfonapht-1-yl)azo]-1-naphtalène-sulfonique (C.I. 14,720),
- sel trisodique de l'acide 6-hydroxy-5-[(4-sulfonapht-1-yl)azo]-2,4-naphtalènedisulfonique (C.I. 16,255),
- sel trisodique de l'acide 3-hydroxy-4-[(4-sulfonapht-1-yl)azo]-2,7-naphtalènedisulfonique (C.I. 16,185),
- sel disodique de l'acide 8-amino-1-hydroxy-2-(phénylazo)-3,6-naphtalènedisulfonique (C.I. 17,200),
- sel disodique de l'acide 5-(acétylamino)-4-hydroxy-3-[(2-méthylphényl)azo]-2,7-naphtalène disulfonique (C.I. 18,065),
- sel disodique de l'acide 2-(3-hydroxy-2,4,5,7-tétraiododibenzopyran-6-on-9-yl)-benzoïque (C.I. 45,430),
- N-[6-(diéthylamino)-9-(2,4-disulfophényl)-3H-xanthène-3-y(iden]-N-éthylethanammonium hydroxyde, sel interne, sel sodique (C.I. 45,100),
- sel disodique de l'acide 8-[(4-(phénylazo)phényl)azo]-7-naphtol-1,3-disulfonique (C.I. 27,290),
- sel disodique 2',4',5',7'-tétrabromo-3',6'-dihydroxy spiro [isobenzofurane-1(3H),9'-[9H] xanthène]-3 (C.I. 45,380),
- sel disodique 2',4',5',7'-tétrabromo-4,5,6,7-tétrachloro-3',6'-dihydroxyspiro[isobenzofuran-1(3H),9'[9H]xanthène]-3-one (C.I. 45,410),
- sel disodique 3',6'-dihydroxy-4',5'-diiodospiro[isobenzofuran-1 (3H),9'(9H)-xanthène]-3-one (C.I. 45425),
- sel sodique de l'acide 2-hydroxy-3-((2-hydroxynapht-1-yl)azo)-5-nitrobenzènesulfonique (C.I. 15,685),
- sel sodique de l'acide 3-hydroxy-4-(3-méthyl-5-oxo-1-phényl-4,5-dihydro-1H-pyrazol-4-ylazo)-naphtalène-1-sulfonique, complexe chrome (Acid Red 195),
- sel de calcium de l'acide 3-hydroxy-4-[(4-méthyl-2-sulfonphényl)azo]-2-naphtalènecarboxylique (C.I. 15,850:1),
- sel disodique de l'acide 3-[(2,4-diméthyl-5-sulfophényl)azo]-4-hydroxy-1-naphtalène sulfonique (C.I. 14,700),
- sel disodique 1,4-bis[(2-sulfo-4-méthylphényl)amino]-9,10-anthraquinone (C.I. 61,570),
- bis[4-(diméthylamino)phényl]-(3,7-disulfo-2-hydroxynapht-1-yl)carbénium sel interne, sel sodique (C.I. 44,090),
- sel interne de bis [4-(diéthylamino)phényl] (2,4-disulfophényl)carbénium, sel de sodium (2:1) (C.I. 42,045),
- bis[4-(diéthylamino)phényl]-(5-hydroxy-2,4-disulfophényl)-carbénium-sel interne, sel de calcium (2:1) (C.I. 42,051),
- N-[4-[(2,4-disulfophényle)[4-[éthyl(phénylméthyl)amino)phényl]méthylène]-2,5-cyclohexadiène-1-ylidène]-N-éthylbenzèneméthanaminium hydroxyde, sel interne, sel sodique (C.I. 42,080),
- sel disodique de (2-sulfophényl)di[4-(éthyl((4-sulfophényl)méthyl)amino)phényl]-carbénium bétaïne (C.I. 42,090),
- acide 1-amino-4-(phénylamino)-9,10-anthraquinone-2-sulfonique (C.I. 62,055),
- sel sodique de l'acide 1-amino-4-(cyclohexylamino)-9,10-anthraquinone-2-sulfonique (C.I. 62045),
- sel disodique de l'acide 2-(1,3-dihydro-3-oxo-5-sulfo-2H-indol-2-ylidène)-2,3-dihydro-3-oxo-1 H-indol-5- sulfonique (C.I. 73,015),
- 9-(2-carboxyphényl)-3-[(2-méthylphényl)amino]-6-[(2-méthyl-4-sulfophényl)amino]xanthylium-sel interne, sel sodique (C.I. 45,190),
- sel sodique de 1-hydroxy-4-[(4-méthyl-2-[(4-méthyl-2-sulfophényl)amino]-9,10-anthraquinone (C.I. 60,730),
- bis[3-nitro-4-[(4-phénylamino)-3-sulfo-phénylamino]-phényl]-sulfone (C.I. 10,410),
- sel disodique de l'acide 5-amino-4-hydroxy-6-[(4-nitrophényl)-azo]-3-(phénylazo)-2,7-naphtalène disulfonique (C.I. 20,470),
- complexe de chrome de l'acide 3-hydroxy-4-[(2-hydroxynapht-1-yl)azo]-7-nitro-1-naphtalène sulfonique (3:2) (C.I. 15,711),
- sel tétranate de 4-(acétylamino)-5-hydroxy-6-[(7-sulfo-4-[(4-sulfophényl)azo]napht-1-yl)azo]-1,7-acide naphtalènedisulfonique (C.I. 28,440),
- 3',3",5',5"-tétrabromophénol sulfophthaléine (bleu de bromophénol), 3,4,5 ,6,3',3",5',5"-octabromophénol sulfophthaléine (bleu de tétrabromophénol),
- mélanges des colorants susmentionnés.

8. Agent de traitement capillaire selon l'une quelconque des revendications 1 à 7, **caractérisé en ce que** la composition aérosol contient, par rapport à son poids, de 0,1 à 10 % en poids, de préférence de 0,5 à 7,5 % en poids, de manière préférée de 0,75 à 5 % en poids, de manière plus préférée de 1 à 4 % en poids, de manière encore plus préférée de 1,1 à 3 % en poids et en particulier de 1,2 à 2 5 % en poids, des agents humectants du groupe propanediol, glycérol, polyéthylène glycol(s), butylène glycol, sorbitol ou leurs mélanges.

9. Agent de traitement capillaire selon l'une quelconque des revendications 1 à 8, **caractérisé en ce que** la composition aérosol contient, par rapport à son poids, de 0,5 à 7,5 % en poids, de préférence de 0,75 à 5 % en poids, de manière préférée de 1 à 4 % en poids, de manière plus préférée de 1,1 à 3 % en poids et en particulier de 1,2 à 2,5 % en poids de talc.

10. Agent de traitement capillaire selon l'une quelconque des revendications 1 à 8, **caractérisé en ce que** la composition aérosol contient, par rapport à son poids, de 0,1 à 10 % en poids, de préférence de 0,5 à 7,5 % en poids, de manière préférée de 0,75 à 5 % en poids, de manière plus préférée de 1 à 4 % en poids, de manière encore plus préférée de 1,1 à 3 % en poids et en particulier de 1,2 à 2,5 % en poids de polymère de renforcement choisi parmi
- les polymères non ioniques à base de monomères éthyléniquement insaturés, en particulier parmi
- les homopolymères de N-vinylpyrrolidone, copolymères non ioniques de N-vinylpyrrolidone,
- les homopolymères et copolymères non ioniques de N-vinylcaprolactame,
- les copolymères de (méth)acrylamide,
- l'alcool polyvinylique, l'acétate de polyvinyle,
- le chitosan et les dérivés du chitosan,
- les dérivés cationiques de la cellulose,
- les copolymères cationiques de 3-(C₁ à C₆)-alkyl-1-vinyl-1-imidazolinium,
- les homopolymères et copolymères contenant l'unité de structure de formule (M-1) 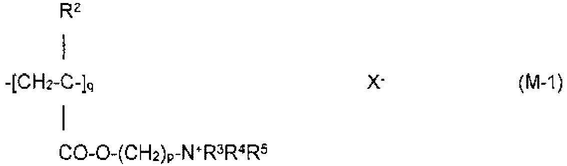 dans laquelle R² = -H ou -CH₃, R³, R⁴ et R⁵ sont chacun indépendamment choisis parmi les groupes alkyle en C₁ à C₄, alcényle en C₁ à C₄ ou hydroxyalkyle en C₂ à C₄, p est 1, 2, 3 ou 4, q est un nombre naturel et X- est un anion organique ou inorganique physiologiquement acceptable,
- les polymères anioniques ayant des groupes carboxylate et/ou sulfonate,
- les polyuréthanes anioniques.
